(19) Europäisches Patentamt
European Patent Office
Office européen des brevets

(11) **EP 3 776 561 B1**

(12) **EUROPEAN PATENT SPECIFICATION**

(45) Date of publication and mention of the grant of the patent:
**19.11.2025 Bulletin 2025/47**

(21) Application number: **18724345.6**

(22) Date of filing: **05.04.2018**

(51) International Patent Classification (IPC):
***G06F 18/2135*** *(2023.01)* ***G06F 18/2411*** *(2023.01)*
*G16C 20/70* *(2019.01)* *G01J 3/28* *(2006.01)*

(52) Cooperative Patent Classification (CPC):
**G16C 20/20; G06F 18/2135; G06F 18/23213; G06F 18/2411;** G01J 3/28; G06F 2218/12; G16C 20/70

(86) International application number:
**PCT/PT2018/050012**

(87) International publication number:
**WO 2019/194693 (10.10.2019 Gazette 2019/41)**

(54) **SPECTROPHOTOMETRY METHOD AND DEVICE FOR PREDICTING A QUANTIFICATION OF A CONSTITUENT FROM A SAMPLE**

SPEKTROPHOTOMETRISCHES VERFAHREN UND VORRICHTUNG ZUR VORHERSAGE EINER QUANTIFIZIERUNG EINES BESTANDTEILS AUS EINER PROBE

PROCÉDÉ ET DISPOSITIF DE SPECTROPHOTOMÉTRIE DESTINÉS À PRÉDIRE UNE QUANTIFICATION D’UN CONSTITUANT À PARTIR D’UN ÉCHANTILLON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.02.2021 Bulletin 2021/07**

(73) Proprietor: **INESC TEC - Instituto de Engenharia de Sistemas e Computadores, Tecnologia e Ciência**
**4200-465 Porto (PT)**

(72) Inventor: **DA COSTA MARTINS, Rui Miguel**
**4200-465 Porto (PT)**

(74) Representative: **Patentree**
**Edificio Net**
**Rua de Salazares, 842**
**4149-002 Porto (PT)**

(56) References cited:
**EP-A1- 1 967 846** **EP-A1- 1 992 939**
**EP-A1- 3 136 270** **EP-A2- 0 807 809**

- **RAMIREZ-LOPEZ L. ET AL: "The spectrum-based learner: A new local approach for modeling soil vis-NIR spectra of complex data", GEODERMA, ELSEVIER, AMSTERDAM, NL, vol. 195, 22 January 2013 (2013-01-22), pages 268 - 279, XP028972744, ISSN: 0016-7061, DOI: 10.1016/J.GEODERMA.2012.12.014**
- **BÖHM G. ET AL: "Quantitative analysis of protein far UV circular dichroism spectra by neural networks", PROTEIN ENGINEERING, DESIGN AND SELECTION, vol. 5, no. 3, 1 April 1992 (1992-04-01), GB, pages 191 - 195, XP055549832, ISSN: 1741-0126, DOI: 10.1093/protein/5.3.191**
- **BATHEN T. F. ET AL: "Quantification of plasma lipids and apolipoproteins by use of proton NMR spectroscopy, multivariate and neural network analysis", NMR IN BIOMEDIC, WILEY, LONDON, GB, vol. 13, no. 5, 1 August 2000 (2000-08-01), pages 271 - 288, XP008105223, ISSN: 0952-3480, [retrieved on 20000621], DOI: 10.1002/1099-1492(200008)13:5<271::AID-NBM646>3.0.CO;2-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 776 561 B1

- O'CONNELL M-L. ET AL: "Classification of a target analyte in solid mixtures using principal component analysis, support vector machines, and Raman spectroscopy", VISUAL COMMUNICATIONS AND IMAGE PROCESSING; 20-1-2004 - 20-1-2004; SAN JOSE,, vol. 5826, no. 1, 1 January 2005 (2005-01-01), pages 340 - 350, XP002444469, ISBN: 978-1-62841-730-2, DOI: 10.1117/12.605156

## Description

### Technical field

The present disclosure relates to spectrophotometry method and device for predicting a quantification of a constituent from a sample to be quantified.

### Background

Spectroscopy is an indirect measurement of metabolites, either for their identification or quantification. Each molecule or atom has a characteristic spectral fingerprint obtained by absorvance or emission, reflectance, fluorescence, phosphorescence and Raman scattering; and band intensities are directly proportional to the specimens concentration.

In pure substances or in simple mixtures, the spectrum carries little interference. In these cases, specimens identification is directly applicable by band matching and intensity is proportional to concentration.

In more complex mixtures, such as, chemical or pharmaceutical products, the spectrum signal is the result of band interference of primary absorvance bands and overtones; resulting into a continuous spectra of overlapping bands. The increased interference between constituents, turns the possibility of quantification by peak intensities difficult. In this circumstances, metabolites must be preferably quantified by their interference pattern (Geladi and Kowalski:1986, Phatak and Jong:1997).

Moreover, depending on the spectroscopy technology, band resolution and spectrum convolution is significantly different, and therefore, spectral information is locally distributed by the recurrent convolution of optical parts, leading in extreme cases of low quality, to a highly auto-correlated signal. The spectrum carries both physical and chemical information, as well as, the complex interference pattern between their constituents. The quantum nature of spectroscopy means the information about any pure compound is widespread along different wavelengths at several scales of intensity. Due to the wave nature of light, superposition of information results into constructive or destructive interference between the sample constituents bands. Therefore, the observed variations body fluids is highly non-linear with local chaotic variations, that cannot simply be modelled by state-of-the-art chemometrics, machine learning or artificial intelligence methods. In order to avoid developing a theoretical support for extracting spectral information from non-linear effects, many chemometritians tried to apply machine learning algorithms such as artificial neural networks, kernel methods and support vector machines. It was expected that more complex model structures could capture all the non-linearity and provide better predictions.

Previous methods present the following difficulties in spectroscopy modelling.

Big data spectral variability - Deep ANN and non-linear SVM are complex function models that fit to all data, That is, a structured monolithic model is produced for a population of data. Such leads to highly complex architectures, which is not the best for big data in spectroscopy, due to the local chaotic nature of the signal. Once a new sample with new local variations is introduced, the model is unable to find the correct co-variance between spectral bands and composition. Furthermore, if not fed with a large amount of data, ANN and SVN are extremely exposed to significant bias in their predictions. These methods became only interesting when the feature space is almost totally represented; which is hard, as biological variability is extremely vast.

Re-training computational cost - As ANN and SVM are global methods, once a new set of outliers are identified, the complex model structure must be re-optimized. Once this is made with large databases, significant computing resources must be used to re-compute model structure (Huangetal:2015).

Outliers detection - The complex structure of ANN and SVN makes difficult to determine 'a priori' if a new spectra is an outlier. As there is no apparent law from which to draw conclusions about the predictability of any new result, knowing if a spectra measurement is an outlier is difficult. This is specially critical in medical, veterinary or even hazardous industrial processes where substances accurate quantification is paramount and prediction failure has disastrous consequences.

Therefore, information processing technologies that take into account the systematic variation of optics and spectroscopy are more likely to solve the problem without the need of high computational cost. For instance, local calibration approaches were developed to breakdown the global spectral variance into characteristic groups where variations are systematic (Ramirez-Lopesetal:2013). In many cases, local approaches outperform ANN and SVM (Solomatineetal:2008). Techniques such as locally weighted partial least squares (LW-PLS) (Naesetal:1990, ChristyandDyer:2006), LOCAL (Shenketal:1997), locally biased regression (FearnandDavies:2003) and CARNAC (DaviesandFearn:2006) and local PLS modeling approaches (Gogeetal:2012) provided complexity reduction and stable calibrations.

One of the latest developments is the method 'spectral based learner' (SBL) used to model big data of NIR soil composition (Ramirez-Lopezetal:2013). SBL is based on a knowledgebase constructed using optimized principal components (oPC) (Ramirez-Lopezetal:2013), where the local calibration is obtained by using the oPC's dimensional distance neighbors (e.g. determined for instance by k-nearest neighbors or other distance metrics), determined by similarity in chemical composition, using the root square mean difference (RSMD) of composition (Ramirez-Lopezetal:2013). Local sample selection is solely based on the effects of major components, that is, substances that influence the spectral fingerprint with lower frequency or signal baseline. SBL will always struggle to quantify lower concentrations, where the information is present in small scales of variation of the spectrum.

The present state-of-the-art approaches are unable to technically solve the complexity of spectrum quantification and provide the necessary accuracy and precision (bias and variance) to be used in critical applications, such as, medicine. Correct medical decisions can only be supported by analytical grade data. The present disclosure presents a method and device intended to overcome the mentioned technical problems and the current technical difficulties of artificial intelligence and pattern recognition in spectroscopy, to provide accurate quantification and classification of spectral samples under complex variability and multi-scale interference.

## Summary of the Invention

The invention is defined in the appended claims, in particular independent claims.

## Summary of Related Disclosure

This disclosure relates to a big data self-learning artificial intelligence method and device for the accurate quantification of metabolites classification of health conditions from spectral information, where complex biological variability and multi-scale spectral interference is present. In particular, this disclosure allows the breakdown of highly complex biological spectral signals into high dimensional feature space where local features of each sub-space are accurately correlated with both a specific metabolite concentration or categorical condition. Such is achieved by a self-learning method, that requires no human intervention. The developed artificial intelligence is able to establish its own knowledgebase when new data is fed by performing feature space transformations, searching directions of co-variance and optimizing local composition-spectral correlations.

These methods allow to establish knowledge maps of both quantifications and classifications, that can be cashed for higher computational performance. In particular, direct search comprises of finding across the feature space data and dimensions that allow a direct linear correspondence between metabolic composition and spectral bands variance. Moreover, a similar approach is derived for defining the convex hull regions of different class of health conditions from body fluid spectra. Such results in the creation of knowledge maps for both quantification and classification.

The present disclosure also allows evaluating 'a priori' the predictability, accuracy and precision of new estimates. Furthermore, this disclosure provides a self-learning approach to de definition of the global feature space using big data, for its correct characterization under high variability, accurate detection of local anomalies, as well as, outliers that can contaminate the knowledge base.

This disclosure is applicable to all regions of the electro-magnetic spectra used in spectroscopy analysis (x-ray, uv, vis, nir, ir, far-ir and microwaves), or with any other type of spectroscopy (absorvance, reflectance, fluorescence, phosphorescence, Raman scattering) where complex multi-scale interference and biological variability is present. It further extends to fields of non-destructive, non-invasive spectroscopy applications in fields such as healthcare, veterinary, biotechnology, pharmaceutical, food and agriculture.

It is disclosed a spectrophotometry method for predicting a quantification of a constituent from a sample to be quantified,
comprising the steps of:

obtaining an electromagnetic spectrum from said biological sample;
projecting said obtained spectrum into a sample point of a multiple dimension vector space associated with feature vectors, herewith a feature space, defined by a predetermined vector basis, wherein each of said dimensions is a prediction feature;
selecting, if existing, a minimum of neighbouring sample points from sample points within said feature space, said sample points having been projected from previously obtained spectra each with a known constituent quantity, such that said minimum maximises the covariance of the projected spectrum of said sample to be quantified together with the projected spectra of the selected neighbouring sample points;
predicting the quantification of the constituent from the sample to be quantified by correlating the known constituent quantity from the selected neighbouring sample points taking into consideration the projected spectrum of said sample to be quantified and the projected spectra of said selected neighbouring sample points.

An embodiment comprises, for determining the predictability of quantification of the constituent of the sample to be quantified, by:

calculating a normal distribution of the prediction error of the constituent quantity from the selected neighbouring sample points;
obtaining a p-value from said calculated normal distribution and from the projected spectrum of said sample to be quantified;
using the obtained p-value as the predictability of quantification of the constituent of the sample to be quantified.

An embodiment comprises, if the minimum of neighbouring sample points is not existing, the steps of: flagging that prediction of the quantification of the constituent from the sample to be quantified is not possible.
An embodiment comprises:

placing the obtained spectrum from said biological sample into a quarantine database;
receiving a measured quantification of the constituent from the sample to be quantified;
accumulating in said quarantine database a plurality of obtained spectra from biological samples to be quantified and respective measured quantifications of the constituent from samples to be quantified;
determining if a subset of the accumulated spectra and measured quantifications in said quarantine database allows predictability of quantification of the constituent of the sample to be quantified;
if the subset allows predictability, releasing the subset from the quarantine database for use in the present spectrophotometry method for predicting a quantification of a constituent.

An embodiment comprises, for determining if a subset of the accumulated spectra and measured quantifications allows predictability of quantification of the constituent of the sample to be quantified:

on placing the obtained spectrum from a biological sample into the quarantine database; and
on receiving a measured quantification of the constituent from the sample to be quantified corresponding to the biological sample;
the steps of:

projecting said obtained spectrum into a sample point of a multiple dimension vector space associated with feature vectors, herewith a feature space, defined by a predetermined vector basis, wherein each of said dimensions is a prediction feature;
selecting, from said quarantine database, if existing, a minimum of neighbouring sample points from sample points within said feature space, said sample points having been projected from previously obtained spectra each with a known constituent quantity, such that said minimum maximises the covariance of the projected spectrum of said sample to be quantified together with the projected spectra of the selected neighbouring sample points of said quarantine database;
predicting the quantification of the constituent from the sample to be quantified by correlating the known constituent quantity from the selected neighbouring sample points from said quarantine database taking into consideration the projected spectrum of said sample to be quantified and the projected spectra of said selected neighbouring sample points of said quarantine database;
determining the predictability of quantification of the constituent of the sample to be quantified;
if the predictability is deemed above a predetermined threshold, releasing the selected neighbouring sample points from said quarantine database for use in the present spectrophotometry method for predicting a quantification of a constituent.

In an embodiment, said released selected neighbouring sample points constitute a local model.
In an embodiment, selecting the minimum of neighbouring sample points from sample points within said feature space, comprises the steps of:

projecting an obtained spectrum into a sample point of the multiple dimension vector space, herewith a feature space;
defining a plurality of search directions in said feature space;
defining a plurality of directional search volumes contained within said feature space, each directional search volume being defined as a region of the feature space that includes said projected spectrum sample point, that extends along a search direction by a predetermined search radius distance from said projected sample point, and that extends from said search direction by a predetermined search width distance;
calculating for each search direction a plurality of corresponding prediction models, wherein each said model is calculated by selecting a dimension subset from the dimensions of the feature space, said model being calculated using the projected sample points within the directional search volume corresponding to the search direction such that covariance is maximised;

selecting the search direction that has the corresponding prediction model that has a maximum predictability of quantification of the constituent to be quantified;
using the projected sample points within a selected directional search volume corresponding to the selected search direction as the selected minimum of neighbouring sample points.

In an embodiment, each directional search volume is defined as a region of the feature space that originates from said projected spectrum sample point.
An embodiment comprises:
minimizing the selected directional search volume by reducing the predetermined search width distance such that the predictability of quantification of the constituent to be quantified is maximized by the model calculated by the selected dimension subset and calculated using the projected sample points within the directional search volume being minimized.
In an embodiment, the prediction model of each search direction is calculated by:

defining a covariance matrix between the feature space and the quantification of the constituent;
minimizing the number of eigenvectors extracted from the covariance matrix that minimizes prediction error;
selecting those eigenvectors that correspond to said minimum;
using a multivariate linear prediction model defined by the selected eigenvectors as the calculated prediction model of each search direction.

In an embodiment, the calculated multivariate linear prediction model joined with the projection defined by the predetermined vector basis provides an interpretive correlation between an input spectrum and constituent quantification.
In an embodiment, each said directional search volumes is a multidimensional box or cylinder defined by a predetermined distance from a line defined by the respective search direction and the projected spectrum sample point.
An embodiment comprises:
filtering orthogonally the variation of the quantification of the constituent to be quantified calculated by the corresponding model of the selected directional search volume in respect of the projected sample points within the selected directional search volume.
An embodiment comprises repeating a selection the minimum of neighbouring sample points from sample points within said feature space, by the steps of:

defining a plurality of search directions in said feature space;
defining an plurality of directional search volumes contained within said feature space, each directional search volume being defined as a region of the feature space that originates from the end of the predetermined search radius distance along the selected search direction of the previously selected directional search volume;
calculating for each said search direction a plurality of corresponding prediction models, wherein each said model is calculated by selecting a dimension subset from the dimensions of the feature space, said model being calculated using the projected sample points within said directional search volume corresponding to the search direction;
selecting said search direction that has the corresponding prediction model that has a maximum predictability of quantification of the constituent to be quantified;
using the projected sample points within a selected directional search volume corresponding to said selected search direction as the selected minimum of neighbouring sample points.

An embodiment comprises repeating the steps above until a predetermined criteria is reached in respect of covariance of the projected spectrum of the sample to be quantified together with the projected spectra of the selected neighbouring sample points.
An embodiment comprises:

repeating the selection the minimum of neighbouring sample points from projected sample points within said feature space, recursively calculating said prediction models;
aggregating said calculated prediction models into an aggregated prediction model, herewith a path model.

In an embodiment, said path model is cached for subsequent predictions of constituent quantification without recalculating prediction models.
In an embodiment, the quantification to be predicted is a logistic function of a class to be determined from a constituent or constituents from the sample.
In an embodiment, the predetermined search radius distance, the predetermined search width distance, and the number of the plurality of search directions in the feature space is determined using an iterative optimization method, in particular a simplex algorithm.

In an embodiment, selecting a minimum of neighbouring sample points from sample points within said feature space comprises selecting a minimum number of sample points above a predetermined threshold of covariance.

In an embodiment, the predetermined vector basis is an orthogonal information-preserving decomposition into constituent functions or into a matricial factor decomposition, in particular singular-value decomposition - SVD, wavelets, Fourier transform, wavelets, or curvelets.

In an embodiment, the pre-processing of the obtained spectra comprises deconvolution and/or resolution enhancing of said spectra.

In an embodiment, the sample is biological and the constituent or constituents are biological metabolites, in particular blood metabolites.

It is also disclosed a non-transitory storage media including program instructions for implementing a spectrophotometry method for predicting a quantification of a constituent from a sample to be quantified, the program instructions including instructions executable to carry out the method of any of the disclosed embodiments.

It is also disclosed a spectrophotometry device for predicting a quantification of a constituent from a sample to be quantified, the device comprising an electronic data processor configured for carrying out the spectrophotometry method of any of the disclosed embodiments.

An embodiment comprises a spectrophotometer and non-transitory storage media including program instructions for implementing said spectrophotometry method.

## Detailed Description

Obtaining accurate quantifications $\mathbf{Y}$ from spectral knowledgebase $\mathbf{X}$ using a projection model $\mathbf{Y} = f\{\mathbf{X}\}$ becomes feasible if: i) the co-variance is stable ($\mathbf{X^tY}$); ii) the variance of the spectral feature space is stable ($\mathbf{X^tX}$); iii) the bias-variance of the predicted $\hat{\mathbf{Y}}$ is low; iv) extracted eigenvectors, projections and coefficients are statistically coherent and interpretable.

Globally stable $\mathbf{X^tY}$ and $\mathbf{X^tX}$ across the feature space of big data biological spectra does not exist. Co-variance direction is non-linear and eigenvectors rotate across the feature space, depending on local characteristics. Given the unlimited number of possible observations of **X,** it becomes unfeasible to quantify a new unknown spectrum $\mathbf{X_{new}}$ if the variance of the feature space is non-linear across the feature space.

Given such physical constrains, it is disclosed in this disclosure a method regarding spectral prediction, based on the fact that any unknown spectra $\mathbf{X_{new}}$ should be consistent with the feature space at a local sub-space, so that, it can also hold consistent information in terms of co-variance with compositional data ($\mathbf{X^tY}$). The prediction of $\hat{\mathbf{Y}}$ is now a problem of finding a consistent sub-space of $\mathbf{X^tX}$ that holds correspondent information about $\mathbf{Y}$, so that $\mathbf{X^tY}$ is consistent with $\mathbf{X_{new}}$ variation, producing a stable and reliable prediction. Moreover, only by ensuring that $\mathbf{X^tX}$ and $\mathbf{X^tY}$ are locally coherent, allows us to know 'a priori' if any unknown spectra $\mathbf{X_{new}}$ can be predicted based on previous knowledge.

One can consider that, there is no 'a priori' model to quantify substances for a given unknown $\mathbf{X_{new}}$, as it happens with previous methodologies (PCR, PLS, LS-SVM, ANN and Deep Learning). It is possible to postulate that for any given $\mathbf{X_{new}}$, there will be a subset of the knowledgebase feature space that will be able to sustain consistency to predict $\hat{\mathbf{Y}}$. Therefore, once a new spectra is recorded, the AI must learn if any subspace in the knowledbase exists that allows to perform a correct prediction according to improve standards.

There are significant advantages of using subspace identification in spectroscopy: i) interpretation of the subspace becomes feasible due to complexity reduction; ii) local independence of data representativity (number of data), that is, predictions became not affected by more data being in the knowledbase; iii) local multi-scale consistency; iv) interpretation of bands used to perform the quantification; iv) better control of what bands are used in quantification; v) spectral corrections are more accurate, as baseline, mie and Rayleigh scattering corrections enhance spectral bands variation if spectral variance is consistent; vi) feature space transformation (e.g. kernel, derivates, wavelets) between locally consistent; and vii) adaptability: as quantification is self-learned, local adaptation will always find the optimal set of spectra **(X)** in the knowledbase that provides the best prediction of $\hat{\mathbf{Y}}$. Sub-space identification allows the AI to self-learn and became independent from human supervision during model building.

The problem of quantification based on complex spectral information is empirically explained in **Figure 1. Figure 1a** shows a collection of spectra, where the composition is a mixture of the same components in different quantities. The substances present in the mixture are highly interference between each other, making impossible to derive directly a peak correlation with concentration to provide a simple method of quantification. Nevertheless, there are four classes of spectral non-linear variation, that is, four modes that may provide direct correlation to the composition of a particular substance. Even under such a simple example, the use of a GLM (e.g. PLS) would provide the prediction of $\hat{\mathbf{Y}}$ with high variance. Moreover, if any mode of variation has lack of representation, providing a prediction for a new spectra within this class, will inevitably provide a high biased prediction. **Figure 1b** shows why euclidean distances are not a good measure of spectral features nor able to correlate to composition. All four groups of variation of **Figure 1a** exhibit completely different non-linear projections that allow quantification that may not be linearly correlated to concentration. Spectroscopy quantification in complex mixtures is non-linear search for co-variation projection of eigenvectors that locally produce minimal bias-variance of predictions.

Therefore, in order to provide accurate quantifications for a given $\mathbf{X_{new}}$ one must search across the feature space the set of neighbors that allow optimal projections, as presented in **Figure 1d.**

**Figure 2a** shows that when using clustering techniques, such as, hierarchical clustering, k-nearest neighbors (KNN) used in the previous art (spectral based learner), it will always result in sub-optimal projections with bias and possible outliers. **Figure 2b** shows the optimal projections for unknown spectra **#1** and **#3,** which are under different local co-variations, and **#2** is an outlier that cannot be predicted by the knowledge base.

Herein, it is disclosed a self-learning method and device for spectroscopy big data. The new method is able to find the coherent eigenvectors of quantification that sustain a consistent $\mathbf{X^tX}$ and $\mathbf{X^tY}$. The proposed self-learning does not produces a monolithic model. For each new data, the system has to learn the coherence of $\mathbf{X^tX}$ and $\mathbf{X^tY}$, to project $\mathbf{X_{new}}$ and estimate $\hat{\mathbf{Y}}$. Moreover, if both $\mathbf{X^tX}$ and $\mathbf{X^tY}$ are locally coherent, the prediction problem of any $\mathbf{X_{new}}$ can be estimated 'à priori'. Metrics about the variance-covariance consistancy allows to infere the local confidence in predictability.

Embodiments of the disclosed method comprise the following three major steps: i) local geometry and sub-space identification - where the local geometry of spectral information is extracted as a characteristic sub-space with characteristic eigenvectors that support local quantification/classification; ii) building the knowledgebase of non-linear feature mapping - process by which applying recursive local geometry and subspace identification allows to build the artificial intelligence knowledgebase on non-linear mapping of spectral information; iii) local optimization of spectral information - process of local refining the quantification or classification by minimizing the local convex hull volume and prediction error by filtering out non-related information in both **Y** and **X,** or their correspondent feature space transformation **K** and **F.**

The following pertains to local geometry and sub-space identification. As presented previously, it is postulated that there is always a local direction clustering of data that is able to sustain a coherent eigenvector(s) of quantification. This local cluster represents a local mode of variation within the vast non-linear feature space. Therefore, let one consider the n-dimensional feature space **F,** where the coordinates of the feature space are proportional to linear combinations of spectral features, that are implicitly, correlated to the sample composition. Let it also be assumed that discrete finite directions at a local point of the feature space, represent a mode of variation in the spectra, coherent with a local level of speciemens concentration. Such enables the possibility of extracting a coherent eigenvector from local co-variance ($\mathbf{X^tY}$), between spectral **X** and composition **Y**. Moreover, highly non-linear feature space can be locally characterized by a hyper dimensional polytope that has consistent directions of quantification, that is, all the data contained by its correspondent convex hull presents a mode where all spectra inside it follow a mode of variation that quantifies different parameters with low bias-variance.

Therefore, the local geometry is directly related to composition at this local subspace, one can find an optimal eigenvector of quantification. The problem of big data spectral quantification and classification is reduced the search of local geometry that: i) minimizes the number of directions/dimensions of the polytope; ii) obtains a principal direction that minimizes bias-variance; and iii) minimizes the convex hull volume of the selected optimal directional polytope; so that a direct linear model is applicable to this finite space approximation.

**Figure 3** illustrates the problem in the feature space with a feature space map. In this example. a 2-dimensional feature space is presented with highly non-linear variation of different classes that occupy different regions of space. The continuous line represents a coherent co-variance feature with a specimen concentration, that is, along this line, is possible to find coherent eigenvectors of local $\mathbf{X^tY}$ in order to produce low error estimates of $\hat{\mathbf{Y}}$. Furthermore, the line represents a self-learned characteristic of the feature space. For instance, every new spectra $\mathbf{X_{new}}$ that is projected into the vicinity of this line can be directly predicted by the self-learned subspace model.

The self-learning process focuses in searching the coherent polytopes subspaces that allow specimens quantification with low bias and variance, being illustrated by **Figure 3,** and with pseudo-code of the process present in **Algorithm 1** (see also **Figure 17a).** Let one assume that a new $\mathbf{x_i}$ is projected into the feature space. Once projected, the self-learning process has to find nearest neighbors that are within the direction of variation with $\mathbf{x_i}$ in relation to **Y**. Therefore, its necessary to search the convex hull that provides the correct direction in the feature space for quantification. Such convex hull must also present a minimal volume and minimal number of eigenvectors of the local $\mathbf{X^tY}$ that predict $\hat{\mathbf{Y}}$. The following sequence of procedures describe an embodiment the self-learning process:

Step A. Direction finding

Objective: Find the minimum directions and local sub-space geometry of $x_i$.

1. Initialization: i) define a circle area around $\mathbf{x_i}$ projection with a radius of search; ii) define the number of directions; iii) define the dimensions of each direction search.
2. Initial search: i) determine the number of optimal eigenvectors and predict the errors of local models; ii) remove directions that are statistically inconsistent;

3. Prepare new iteration: i) inside the consistent directions, remove the worst contributions, removing search length or increasing in a particular way; ii) taking into consideration the extreme vertices' of each convex hull and $x_i$, compute the new directions of search;
4. The search loop: i) determine the number of eigenvectors and prediction error of the new directions; ii) eliminate the worst directions; iii) re-dimension the search by eliminating the worst (smaller or larger) length, and increase or decrease the search length accordingly; iii) loop the previous operations until no statistically significant direction or dimension change occurs.
5. Output: Minimum number of feasible directions and convex hull volume of each direction.

Step B. Optimization of the convex hull

Objective: minimize the convex hull volume and prediction error

1. Initialization: Merge the previous output data into an initial cluster that defines the initial convex hull.
2. Define: i) the outer vertices' of the convex hull; ii) minimum and maximum moving boundary of the convex hull adaptive geometry.
3. Main loop: i) determine model errors; ii) remove outliers; iii) define the new borders of the convex hull by using simplex geometric optimization - for each outlier removed, move the boundary inwards; iv) compute the new convex hull. Do this cycle until no more outliers are found and model error is stable.
4. Output: optimal convex hull and local model prediction

At the end of this procedure, one expects to obtain the optimal geometry of data that is able do predict any new spectra $\mathbf{x}_i$.. Mathematical and algorithmic details are presented in **Algorithm 1.**

The following pertains to mapping the feature space - building the knowledgebase of quantification. Following a similar philosophy, one can recursively map the self-learning process across all feature space. This mapping constitutes the global knowledge base of the big data spectral data feature space. Let it be taken into consideration all the steps in **Algorithm 1** and apply it recursively across the feature space, following a stepwise sequential protocol as illustrated in **Figure 4.** The procedure is as follows:
Objective: Sequentially mapping (4b) the geometry of co-variance in the feature space (4a).

1. Initialization: i) start at any given point of the feature space; ii) define: search circle diameter, number of search directions and dimensions of search area;
2. Perform Algorithm 1: define the local linear geometry of the convex hull for the spectra $\mathbf{x}_i$
3. Recursive mapping: select inside the optimized convex hull of $x_i$, a new data point $x_{i+1}$ and perform recursively Algorithm 1 until no more directions are feasible to be extracted to expand the convex hull.
4. Re-sample: Proceed to another uncovered location in the feature space
5. Main loop: repeat operations 3 and 4 until a given ratio of coverage of the feature space volume is assured
6. Compilation: Compile knowledgebase quantification paths in the feature space map by registering all model paths to be used as cached models (4c)
7. Output: Compiled mapping of cached models (4c)

Mathematical and algorithmic details of this procedure are presented in **Algorithm 2.** Details of the recursive mapping can also be found in **Figure 17b.**

The result of this process is the construction of the feature space quantification map in **Figure 4c.** The map constitutes the self-learning process of the artificial intelligence method and device. The lines in the map represent coherent path of model local prediction, that is, when a new spectra $\mathbf{X}_{new}$ is projected nearby the line convex hull, will likely follow a similar mode of variation of its neighbors inside the convex hull and can be predicted based on the data of the local model. The characterization of the feature space allows to:

i. Use cashed models to speed-up computing efficiency - if a new spectra is projected into the convex hull of a previous prediction line, the calculation can be performed directly with a cashed model, where calculations are direct;
ii. Characterization of typical conditions that lead to different modes of quantification - many prediction lines provide metabolic information for different types of health conditions and their evolution;
iii. Determine how well the information is represented across the feature space - only regions with sufficient data allow to produce correct quantifications and effective searches;
iv. Provide a map for understanding spectral patterns along time, that is, an interpretation of spectral pattern recognition for the implementation of precision medicine;
v. Provide the basis of a higher level artificial intelligence for condition diagnosis using non-supervised spectral information, allowing the construction of a non-linear classification map of complex and multi factor health conditions.

The following pertains to classification mapping. The self-learning artificial intelligence method for classification has as major objective of finding the class geometry in the feature space by: i) maximizing the local volume of the class; ii) minimizing the total volume across the feature space in the case of non-linear classes; and iii) minimizing the error of class prediction; by delimiting the class boundary with relevant eigenvector variation. Furthermore, one can expect that many classes can be highly non-linear and extremely segmented throughout the feature space. Many classes can also have scattered clusters across the feature space, because other conditions are dominant of feature space variation.

Due to the complex classification of health conditions, and as many conditions are multivariate, the supervised clustering is devised into the following classes: i) single univariate diagnosis - where the discrimination function is a single parameter interval or a threshold; ii) exclusive univariate or multivariate diagnosis - where only isolated cases of each class in the feature space are identified without any overlapping with other classes; and iii) multivariate/complex diagnosis - where only overlapping of data from multiple conditions in the feature space are taken into consideration (see **Figure** 5).

The clustering criteria allow to characterize complex health conditions and map them into the spectral feature space, constituting a classification knowledgebase. The following procedure was developed to build the classification knowledge map: Objective: Sequentially mapping the geometry of classes logistic probability co-variance in the feature space

1. Initialization: a) Define the clustering criteria: a) univariate classes; b) exclusive classes or c) multivariate classes; and d) each class threshold; b) Provide: supervision vector s or matrix S
2. Convex Hull determination: i) select the supervised data in the feature space; ii) find the max and min coordinates of the supervised data in the feature space; iii) select one of the vertices'; iv) define the size of the directional search box; iv) define the volume increment criteria of the convex hull ($\delta$v).
3. Check cluster individuality: If the min and max cannot hold a coherent class prediction, clusters are bounded by information that is not common, and need to be segmented by the minimum global volume criteria, where $v_{optimal} = \max \Sigma_{i=1}^{n} v_{local\ cluster} + \min v_{global}$; If all clusters are segregated, then $\min v_{global} \rightarrow \max \Sigma_{i=1}^{n} v_{local\ cluster}$; if there is only one local cluster: $v_{optimal} = \max \Sigma_{i=1}^{n} v_{local\ cluster}$;
4. Initial search: i) determine the number of eigenvectors and predict the errors of classification; ii) remove statistically inconsistent data; iii) if no relevant direction is found, re-shape the convex hull geometry by moving inwards to a $\delta$v and perform step 4; repeat steps 1 and 2 until it stabilizes.
5. Determine clusters boundary: For each cluster, perform Algorithm 2, where the supervision vector s or matrix S is the logistic function probability of the cluster class associated to the corresponding spectra. See **Figure** 5 how Algorithm 2 is used to find the cluster predictive convex hulls.
6. Compilation: Compile knowledgebase classification clusters in the feature space map by registering their convex hulls
7. Output: Compiled mapping of cached clusters

Mathematical and algorithmic details of this procedure are presented in **Algorithm 3.**

At the end of this procedure, the complete cluster map of the feature space is recorded as a classification knowledgebase. The full composite of all types of classifications for different conditions represent the classification complexity of the knowledgebase, where interactions between conditions, as well as, their metabolic causes can be studied. By projecting a new spectra into this classification map, one can predict the expected probability of a correspondent condition based on the coordinates of the knowledgebase map.

The following pertains to the geometry of local variation. Previous sections explained how the AI of this disclosure is able to provide quantification and classification by recurring to search algorithms across the feature space using co-variance eigenvector extraction, providing maps of quantification and classification. The study of local geometry of variation lies at the heart of the AI disclosure.

Let it be considered that any collection of spectra **X** and compositional data **Y** can be transformed (e.g. kernel, derivative, Fourier, wavelets, curvelets) into the feature space **F** and **K,** respectively. We must find a basis **W and C,** so that, the covariance between local latent variance of **F** and **K, T and U** are maximized. The problem is reduced to the local optimization in the feature space of:

$$f(w,c) = \mathit{argmax}(t^t u)$$

where: $\mathbf{f=tw^t}$; and $\mathbf{k=uc^t}$ and subjected to: $\mathbf{w^tw}$=1 and $\mathbf{c^tc}$=1. By applying the Lagrangian multipliers method to solve the optimization problem, one resumes it to:

$$K^tF = W\Sigma C^t$$

which is the singular value decomposition of $\mathbf{K^tF}$, where $\mathbf{w}$=$\mathbf{W}$[1,], $\mathbf{c}$=$\mathbf{C}$[1,], with associated variance $\Sigma$[1,]. One can further conclude that $\mathbf{F^tKKF^t}$ $\mathbf{w}$ = $\lambda$ **w and** $\mathbf{K^tFF^tK}$ **c =** $\lambda$ **c.** Therefore, **w and c** are characteristic eigenvectors of $\mathbf{Cov(F,K)^2}$ = Cov(**K,F**)$^2$, expressed in the latent space $\mathbf{t^tu}$, where **w and c** spawn a characteristic dimension of the co-variance geometry.

The same kind of derivation is feasible assuming: *f*(**w**,**c**) = *argmax*($t^tu$) when **t=u**, being particularly useful, because, after deflation t becomes a orthogonal. This assumption, is also the basis of other eigenvector extraction algorithms (Indahl:2014).

In order to study the geometry of $\mathbf{t^tu}$, an ortho-normal basis of eigenvectors **w** and c is necessary, so that, for each local **F** one can derive its local characteristic dimensions and geometry. Such is achieved by deflation of **F** and **K:**

$$F_{i+1} = F_i - t_i w_i^t$$

$$K_{i+1} = K_i - u_i c_i^t$$

where: $\mathbf{t_i}$=$\mathbf{F_i w_i}$, $\mathbf{u_i}$=$\mathbf{K_i c_i}$, and $\mathbf{w_i}$=$\mathbf{w_i}/\|\mathbf{w_i}\|$, $\mathbf{c_i}$=$\mathbf{c_i}/\|\mathbf{c_i}\|$. Recurrent deflations until the maximum rank of **F** allow to determine the geometry of co-variance and its complexity, by interpreting $\mathbf{t_i}$, $\mathbf{w_i}$ and their corresponding importance in relation to the captured covariance $\Sigma$ for each eigenvector (Pelletal:2007, Woldetal:2009).

When using the approach where t is orthogonal, deflation is performed as follows:
where **p** and **q** are determined by:

$$F_{i+1} = F_i - t_i p_i^t$$

$$K_{i+1} = K_i - u_i q_i^t$$

$$p_i = F^t_i t_i \, (t_i^t t_i)^{-1}$$

$$q_i = K^t_i t_i \, (t_i^t t_i)^{-1}$$

From the relation of **P** and **Q,** one can derive a direct linear model, such as K=$\mathbf{F}\beta_{pls}$ + e, where:

$$\beta_{pls} = W(P^tW)^{-1}Q$$

where $\beta_{pls}$ is the pls regression coefficients.

The fact that a complex geometry of **T** is condensed into a oblique projection $\beta_{pls}$ (PhatakandJong:1997), and a GLM is produced, is the cause of PLS inefficiency in big data, especially if a relative high number of dimensions or components are used due to a non-linear feature space. Therefore, this strategy implies that the local structure of $\mathbf{K^tF}$ has almost only systematic information about **Y** contained in **X,** with almost no random effects at different scales of the spectroscopy signal. Moreover, the correct feature space transformation is the one that makes possible to obtain similar information structures of $\mathbf{F^tF}$ and $\mathbf{K^tF}$, so that ideally:

$$(K^tK - \lambda_k)\, v_k = 0$$

$$(F^tF - \lambda_f)\, v_f = 0$$

$$(K^tF - \lambda_{kf})\, v_{kf} = 0$$

the local optimization problem remains posed as f(w,c) = *argmax*($t^tu$) but with the ideal restriction of information structure being similar ($\mathbf{v}_k$ ~ $\mathbf{v_f}$ ~ $\mathbf{v}_{kf}$). In perfect conditions, spectral information shares collinear eigenvector structure with the composition, such as it happens with pure compounds or substances with negligible interference. Thus, providing co-variation maximization in the first component is paramount.

A way to provide the AI the interpretation to both t and w, is to make both pairwise orthogonal. We can orthogonalized t using the pls definition that produces orthogonal w:

$$F=TW^t; T=USV^t$$

$$F=USV^tW^t = US(V^tW^t)$$

$$F=T_oW^t{}_o$$

where $\mathbf{T_o} = \mathbf{US}$ and $\mathbf{W}^t{}_o=(\mathbf{WV})^t$ (Ergon:2007, Ergon:2009). There is a direct correspondence to the orthogonal scores $\mathbf{T_w}$ by

$$T_w=T(P^tW)^{-1}$$

And therefore, the AI has a way to perform pattern analysis in orthogonal $\mathbf{T_w}$ or $\mathbf{T_o}$ with corresponding orthogonal **W** in order to derive the coherence of the local subspace and models.

Respecting the following feature space internal relationship:

$$t_i = T_w\ \beta_T$$

given by least-squares estimates:

$$\beta_T = (T_w{}^t\ T_w\})^{-1}\ T_w{}^t\ t_i$$

So that any feature space sample projection into $\mathbf{T_w}$ follows a coherent linear interference pattern for the local relevant eigenstructure. Therefore, any given new data projected into $\mathbf{T_w}$t be contained in the confidence intervals of $\mathbf{t}_i = \mathbf{T_w}\ \boldsymbol{\beta}_\mathbf{T}$,

The complexity of a local dataset can be reduced by refining both samples and variables, as shown in **Figure 6.** Within the local direction selection of **Algorithm 1 one** has to find groups of samples and variables that provide a consistent eigenstructure. Therefore, a fitness function that translates local stability of co-variance is also proposed as the optimization procedure, opposed to the simple use of cross-validation of residuals. The local optimization problem has the following properties: i) blindly select the number of starting datasets; ii) perform relevant PLS regression for each dataset; iii) project into the scores space (**T**=**FW**); iv) use robust linear regression to identify eigenstructures inside **T** (e.g. RANSAC); v) re-do the procedure until a threshold is achieved. However, the existence of a linear model in the T scores space means that deflation is modeling systematic variation within all the data used to build the local linear PLS model.

The consistency of each covariance direction is determined by cross-validation (e.g. leave-n-out), where all data points must sustain the local eigenstructure. For any new unknown data, the prediction is performed as follows: i) determine the consistency of predictions in selected subspace; ii) low bias-variance of all training set; iii) low bias-variance of prediction for the unknown data using the different data in the selected dataset; iv) presence in the linearity of the **T** eigenstructure. Moreover, the predictability of any new unknown data can be obtained by deriving the confidence intervals of the extracted linear eigenstructure in **T,** so that a p-value of the prediction is also forecasted. p-Values above pre-defined thresholds can be considered categorical or unpredictable. The AI has the possibility 'a priori' of knowing if a prediction with the necessary accuracy, because it only uses well known coherent eigenstructured data to perform predictions.

To provide the correct local optimization model one must:

i) minimize the residuals and their deflation structure;
ii) maximize the number of samples and minimizing the number of variables within the same co-variance; iii) ensure coherence inside the **T** space; and iv) eigenstructure similarity between **F** and **K.** Taking all theses objectives into consideration, we can express them with the following optimization function as:

$$J = argmin\left(PRESS \times \left[\frac{n_{pc}}{n} + \frac{n_{sel.vars}}{n_{vars}} + \frac{1}{cov(\mathbf{V},\mathbf{W})} + p - value\right]\right)$$

which can be regarded as a 'corrected' PRESS (Predicted Error Sum of Squares), where: **npc** is the number of components, **n** is the number of data, $\mathbf{n_{sel.vars}}$ is the number of selected variables of **F** from a total of variables $\mathbf{n_{vars}}$, **cov(V,W)** is the covariance of $\mathbf{F^tF}$ and $\mathbf{K^tF}$ eigenvectors, and p-value the probability value of the least squares model in the **T** space. At the end of this optimization, optimal local models are obtained. Further model refinement is performed by orthogonal information filtering.

The following pertains to coherence of the local sub-space. The coherence of the local feature space F is ensured by: i) eigenstructure similarity between F and K; ii) low complexity; and iii) information determinism. F and K have similar eigenstructure when:

$$j = argmax\ (\mathbf{V_k}^t \backslash \mathbf{V_F})$$

where: F = $\mathbf{U_F S_F V_F t}$ = $\mathbf{T_F V_F}^t$; and $\mathbf{K}$ = $\mathbf{U_k S_k V_k}^t$ = $\mathbf{T_k V_k}^t$.

There is an efficient transformation of **X** and **Y**, where **F**=f(**X**) and **K**=f(**Y**), so that ideally $\mathbf{T_K} = \mathbf{T_F}$. As spectral information is multi-scale, one can propose the following multi-scale optimization of signal basis (e.g. Fourier, wavelets, curvelets):

$$F=\Sigma_{i=1}^{z}\ \theta_j \mu_j$$

where i's are the selected individual signal scales so that $\mathbf{V_k}^t\mathbf{V_F}$ is maximized.

Eigenstructure of $\mathbf{K^tF}$ is preferably of extreme importance. Complexity of **F** can be estimated by the distribution of its eigenvalues $\Sigma$, which define the characteristic dimensions of the feature space. In spectroscopy signals, one expects $\Sigma$ to decrease exponentially to a limit value:

$$\Sigma_i = \Sigma_r + (\Sigma_1 - \Sigma_r)\ e^{-ki}$$

where $\Sigma_i$ is the expected i'th eigenvalue, $\Sigma_r$ the residual eigenvalue, $\Sigma_1$ the largest eigenvalue, and **k** the decay factor. Local complexity of the feature space can be measured by the following metrics:

$$C=npc/(k\ n)$$

When $\mathbf{k} \to +\infty$ implies that $\mathbf{C} \to 0$. Such limit is asymptotically approximated when $\mathbf{npc} \to 1$ and $\mathbf{n} \gg \mathbf{npc}$. When $\Sigma_r \to 0$, $\mathbf{K^tF}$ is rank deficient. Randomness of eigenstructure is obtained by randomization of **F, K** and $\mathbf{K^tF}$ (Martinsetal:2007d). Row randomization allows to determine the limit of sample spectra that determine the number of eigenvectors that spawn the row vectors; where as, column randomization determines the limit of eigenvectors that allow variables spawn of column vectors. Statistical stability of the number of eigenvectors is provided by cross-validation.

The following pertains to sub-space Information Optimization. Given the previous procedures, the selected direction of the feature space already provides a stable linear model. It is further expected, that the minimal number of eigenvectors are necessary to predict **Y**. Despite the signal being pre-processed (e.g. baseline, scattering effects, stray light) and transformed to a better feature space basis, there will be always systematic interference in the data. Such interferences affect the scores-loading **(t-p)** relationship beyond the first component. Therefore, ideally relationships should be obtained with only one eigenvector. Such is not always feasible, but, one can greatly simplify model relationships by orthogonal filtering.

**F** and **K** may have systematic information that is not related to each other. Therefore, one must know how the local information is structured, that it how much information **F** and **K** hold in common, and how much is independent by performing orthogonal filtering (TryggandWold:2002, TryggandWold:2003, Bylesjoetal:2008). **Figure 7** shows how orthogonal filtering results into lower complex eigenstructure. **Figure 7a** to **7c** shows how the previous steps optimized the local calibration dataset using **X1, X2** and **X3** subsets, greatly reducing the complexity of the linear model to only three predicting latent variables. Such result still shows that **X1, X2** and **X3** subsets have systematic interference and can be subjected to orthogonal filtering, so that:

$$\mathbf{F=TP^t+T_oP_o{}^t}$$

$$\mathbf{K=TQ^t+U_oP_o{}^t}$$

where **T** are scores with common information between **F** and **K** that maximize co-variance, and **P, Q** the corresponding loadings. $\mathbf{T_o}$ and $\mathbf{U_o}$ the orthogonal scores to the covariance and $\mathbf{P_o}$, $\mathbf{Q_o}$ the corresponding orthogonal loadings; that is, $\mathbf{T_o}$ is orthogonal to **K** and $\mathbf{U_o}$ is orthogonal to **F** (Trygg and Wold:2003).

By recursive selection of samples and variables, one can maximize $\mathbf{TP^t}$ **(Figure 7c** to **7d),** where the number of latent variables is minimized to the optimal lower level near one, that is, no deflation is necessary, and exists a direct correspondence between **F** and **K.** Its expected that the correct feature space transformation leads $\mathbf{T_oP_o^t} \rightarrow 0$ and that $\mathbf{F=TP^t}$ as obtained by regular PLS algorithms.

Similarly, $\mathbf{U_oP_o^t}$ would be zero. Any quantification with analytical grade quality should not have any systematic variation, orthogonal to its quantification. When $\mathbf{U_oP_o^t}$ is significant, it means that the AI cannot be properly trained to provide an accurate prediction, as the original training information suffers of systematic errors. Under the correct conditions, $\mathbf{U_oP_o^t} \rightarrow 0$ and $\mathbf{TP^t}$ >>> $\mathbf{T_oP_o^t}$ so that $\mathbf{K=F\beta_{pls}}$.

If $\mathbf{T_oP_o^t}$ is significant, the feature space transformation was inefficient. In these cases, prediction is performed by applying the orthogonal filter first, $\mathbf{t_o} = \mathbf{fp_o/(p_o^tp_o)}$, and $\mathbf{f_{corr}=f-t_op_o^t}$, and $\mathbf{k=f_{corr}\beta_{pls}}$. For completeness, the method is described in **Algorithm 5** (see also **Figure 17c).**

The following pertains to metrics for sub-space characterization. One of the main advantage of the proposed approach is the possibility of characterization of the self-learned knowledgebase by incorporation of maps of local learning metrics, such as: i) number of data representation; ii) eigenstructure complexity; iii) collinearity between **F** and **K;** iv) predicted sum of squares (PRESS); v) variance of $\mathbf{K^tF}$; and vi) model information structure. Detailed metrics of the knowledgebase are presented in **Table 1.**

By characterizing the feature space, the AI system manages both self-leaning and prediction by knowing how the different regions of the feature space cover the quantification and qualification accurately.

The following pertains to self-learning mechanics. The previous sections demonstrate the algorithms and algebraic procedures of the self-learning method and device. Herein, its provided how the procedures are put together into a system that auto-implements its self-learning from feed data without human intervention, so that it can: i) learn autonomously by data feed from zero data to vast quantities of data in big data spectroscopy data; ii) determine the best multi-scale feature space that best captures co-variance; iii) predict new unknown data based on the knowledgebase and how it handles unpredictable data; iv) self-learn building the quantification and classification maps, and uses them to perform computationally efficient predictions and learning from new data.

Biological variability in body-fluids and body tissues is extremely vast. In big data, one may never determine the meaning of a representative sample to build a robust knowledgebase to be able to build a monolithic model strategy that copes with all the possible spectral combinations. Moreover, biological systems evolve, and their biochemistry is always changing, new cells, new proteins, new metabolites. Therefore, spectroscopy AI applied to biological systems must always self-learn. The developed system is able to self-learn from an initial, very limited, knowledgebase, by constantly adding new data that the system cannot predict. The system begins by computing the feature space and the initial knowledgebase by using the metrics and methods of the previous sections. By managing the predictability of each sub-space of the feature space, the system can sustain if the new acquired data is either predictable or should enter a learning cycle. If cannot be predicted, data is added to a quarantine database, that acts as a vault repository of data that either has no neighbors (e.g. in the beginning, any system will never cover all the feature space) or consistent modeling. The gathered data in the quarantine database passes only to the knowledgebase once new gathered data completes the corresponding area of the feature space allowing the development of a coherent sub-space knowledgebase.

**Figure 8** shows the main mechanics of the self-learning process. Let's consider that the system is initially fed with a limited number of pair of spectra and corresponding composition **X** and **Y**. As any new **X** is recorded, it's projected into the initial feature space map and tested if it belongs to existing knowledbase. If the projection is within the vicinity of an existing model path, and a direct prediction using existing cached model, a prediction is formulated. If the prediction is not within the the expected quality, and if there exists neighbors that make it possible, a new model is built by the **Algorithm 1,** a prediction is performed and the corresponding model and path obtained by **Algorithm 2** are cashed.

When any new spectra is projected into the feature space and has no neigbours, its immediately quarantined. The system enters into the learning cycle and asks the user or system to provide the composition of the sample to be quarantined. Once it has the pair **X** and **Y**, it searches for quarantine neighbors. If it has no neighbors, the data just stays quarantined. If it has neighbors, the learning process begins using **Algorithms 1** and **2** for searching both local models and build the local co-variance map. Only when a new data in conjunction with the quarantined data are able to produce a consistent local model and model path, the data is certified to pass into the knowledgebase. The knowledgebase receives constant updates as new data is added, and predictions are extended to new regions of the feature space.

In this sense, the system: i) never produces predictions that are not within the knowledgebase; ii) maintains and studies the quarantine database; iii) validates quarantine data to pass into certified knowledgebase; iv) only uses certified data to build the knowledgebase and predictions; v) self-learns without human intervention; vi) independent of the data size, growing the knowledgebase with fed data. Moreover, this approach does not need large scale databases for starting building the knowledgebase and performing predictions, such as deep learning neural networks. The system only uses the certified knowledgebase, and therefore, predictions do not suffer from bias, as other modeling approaches would, because they need significant amount of data to produce a globally stable model architecture. Co-variance, classification maps and cached models make the system very computationally efficient. The system turns any spectrometer into an operating independent machine that does not need human intervention to build mathematical models as today's previous-art systems.

Finding the correct basis of transformation of **X** into **F** and **Y** into **K** lies at the heart of building a comprehensive feature space where local linear models are extracted as presented in **section V.** The basic principal for feature space transformation is the maximization of the eigenstructure similarity between **F** and **K.** If a base transformation is able to filter systematic variance unrelated between **X** and **Y** and noise, the eigenstructure of **F** and **K** became equal.

**Figure 9** shows how the feature space transformation is performed. Any spectroscopy signal is decomposed into a orthonormal basis (e.g. Fourier, wavelets, curvelets). These basis provide an independent basis to re-construct scales of the signal based on the basis properties. If present, the information about any metabolite is scattered across different scales of the spectrum, and therefore, the optimal spectral variation for a particular molecule has to be extracted from the original signal using scale reconstruction.

After full spectra decomposition, one must find the optimal basis that provides the combinations that maximize the eigenstructure similarity between **F** and **K,** where $\mathbf{F=T_fV^t_f}$ and $\mathbf{K=T_kV^t}$; and $\mathbf{V^t_kV_f}$ is maximal. Under perfect match of information, $\mathbf{T_f=T_k=T}$, so that both **F** and **K** have the same eigenstructure. Note that, under NIPALS PLS, the assumption forehand that maximizes correlation the non-transformed **X** and **Y** it that part of the information structure has the same eigenstructure, that is the same scores **T.** Here in, we first build a feature space that will allow scores similarity assumption, greatly contributing for the success of the presented disclosure. Its expected that once the feature space transformation is achieved that a direct linear relationship between **K** and **F** exists: $\mathbf{K=F}\beta$. Therefore, what we prove is that PLS or SVM type of assumptions are only possible if the eigenstructure of **K** and **F** are similar. Otherwise, systematic information will contaminate the scores inner relationship assumption and coherence. The same principles can be applied to artificial neural networks or 'deep learning'.

Therefore, let's consider that one decomposes into an orthonormal basis $\mu$ both K and **F:**

$$F = U_f\ \mu$$

$$K = U_k\ \mu$$

where there is a combination of $\mathbf{U_f}$ and $\mathbf{U_k}$ that minimizes the error e of $\mathbf{U_f} = \mathbf{U_k}$, where $\beta = (\mathbf{U_f^t\ U_f})^{-1}\mathbf{U_f^t\ U_k}$. The problem of finding maximal similarity of eigenstructure is an optimization problem of finding the best linear combinations of $\mathbf{U_f}$ and $\mathbf{U_k}$ that maximize the common information between **F** and **K,** defining autonomously the feature space transformation.

By performing this transformation, most of the unrelated systematic and random components of the spectra and composition are eliminated. The system self-learns how to extract the best combinations of $\mu$ that quantify a particular metabolite by evolutionary algorithms, such as, simplex, particle swarn optimization and genetic algorithms. Once a feature space transformation is learned for a particular sub-space, the system does not need to re-calculate, but uses the transformation directly to produce a prediction.

**Figure 10** shows the fluxogram of feature space transformation where: i) the original signals are decomposed; ii) the initial estimate of best basis are estimated by linear regression; and iii) the basis combination is optimized by evolutionary methods. If a combination of basis is found so that the eigenstructure criteria is met, the information about the transformation is cached and used in future perditions as the feature space transformation for building the feature space.

The following pertains to cashed models, co-variance and classification maps. Using cached models, co-variance and classification maps are paramount for computationally efficient self-learning artificial intelligence, leading to significant savings in computational resources. **Figure 11a** shows how cached models are used to speed up predictions. Once a new spectra is recorded, it's projected into the feature space and checked for a model path nearby. If so, the prediction is performed by using methods in section IV; and once any new spectra is recorded the following actions are performed:

i) is a cached model is able to perform the predictions accurately, the result is presented to the end user;
ii) if neighbor models are able to present boundary threshold quality predictions, the system can provide a consensus prediction before computing a new model and updating the knowledgebase;
and iii) if neighbor models do not provide sufficient quality predictions, a new search for a local model is performed, deploying a new model path in the knowledbase.

The following pertains to results and discussion, in particular quantification. Herein, a demonstration the effectiveness of the self-learning artificial intelligence method is performed, by benchmarking the prediction of unknown blood and blood serum samples. Results are compared to the state-of-the-art of chemometrics partial least squares (PLS) global model to provide a simple base of comparison to the previous art. The global PLS was obtained balancing between bias-variance by cross-validation to derive the minimum number of eigenvectors, or latent variables (LV's). Whole blood and serum unknown sample predictions were analyzed in terms of: i) model complexity; ii) average error of prediction (%); and iii) co-linearity - Pearson correlation ($R^2$).

**Figure 13** exemplifies why PLS cannot cope with the complexity of a biological fluid, such as blood. Despite erythrocytes are the major cellular component of blood, and directly related to hemoglobin content, it could be expected that a linear model, would be sufficient to predict accurately the amount of erythrocytes cells. **Figure 13a** shows exactly the opposite, that erythrocytes spectral quantification is highly non-linear affected by significant interferences, so that, a PLS model shows very high variance and significant bias at high erythrocytes count (e.g. > $5 \cdot 10^{12}$ cells/L). Interferences are expressed in the 7 LV's of the PLS model. Such means that non-linear iterative least squares had to deflate 7 eigenvectors to find a common direction in data that quantifies erythrocytes count. This large variance means that even major components exhibit complex spectral patterns, that once reduced to linear quantification, significant prediction bias is obtained (11.50%, **Table 1).** General linear models struggle to achieve analytical grade predictions in healthcare.

**Figure 13c** shows the PLS prediction for leukocytes. Leukocytes are present in blood in lower concentrations than erythrocytes, but are still, a significant proportion of the cellular component. The difference in magnitude is enough to show that it is not possible to predict leukocytes with PLS. Results of **Figure 13c** show that predictions have a very significant variance and large bias. PLS could only provide a model with 27% error ($R^2$=0.45), with a large number of LV's (10), showing the significant amount of spectral interference affects the leucocytes quantification in hole blood.

Erythrocytes and leukocytes are a good example on how the self-learning method handles the complexity of spectral information to provide an accurate prediction based on local multi-scale modeling. **Figures 13b** and **13d** present the self-learning artificial intelligence results for erythrocytes and leukocytes, respectively.

Both parameters exhibit very low variance and bias, allowing medical grade quantification for diagnosis, with only 2.4% and 5.15% error and very significant correlations **(Table 1).**

Most importantly is the complexity reduction of both models to only one LV. The self-learning artificial intelligence was able to find local multi-scale linear relationships, filter variables and samples, so that, a direct correspondence between spectral information and quantification was found, filtering out the complex interference effects in biological samples.

**Table 1** resumes the quantification results for whole blood and blood serum parameters. Hemogram parameters such as erythrocytes, hemoglobin, hematocrit, MCV, MCHC, leukocytes and platelets. Results how a very significant improvement by the self-learning method and device where all parameter estimates exhibit errors below 6% within the studied range.

**Figure 14** shows the results for bilirubin and myoglobin quantification in blood serum. Bilirubin is a significant constituent of blood serum, with yellow-brown coloration. Myoglobin is present in lower quantities, but when present, its spectral fingerprint is very significant in blood serum in the vis-nir region. Therefore, it would also be expected that both molecules could be be linearly quantified by a PLS model. Results in **Figure 14a** and **14c** show that bilirubin and myoglobin PLS prediction exhibits very significant variance, with errors of 12.5% and 31.0%, respectively. Despite these molecules provide a very strong fingerprint in the spectral signal, they still suffer significant interference.

The most relevant result is the fact that bias-variance is significantly reduced when using the self-learning artificial intelligence method of the disclosure **14b** and **14d.** Most models decrease in complexity, and all parameters that are presented in higher concentrations only use 1 eigenvector projection (one LV). The proposed method was able to find a local multi-scale spectral information linearly correlated with molecular quantification. In this sense, all the studied hemogram parameters (erythrocytes, hemoglobin, hematocrit, MCV, MCHC, leukocytes and platelets) were able to attain analytical grade quality with bias below 6%.

Similar conclusions were obtained for blood serum, where high concentration parameter such as bilirubin or high absorvance such as myoglobin are directly quantifiable using only 1 LV. Other lower concentration parameters, such as, glucose, creatinine, CRP, triglycerides, urea and uric acid, greatly reduced their model complexity to 2 to 3 LV's. Such is an indication that lower concentration parameters suffer more interferences and local variations, as well as, their accuracy starts to be affected by the detector background noise.

**Figure 15** presents the benchmark of PLS vs Self-learning artificial intelligence of the present disclosure. PLS modeling could only sustain POC qualitative quantification for: erythrocytes, hemoglobin, MCV, MCHC, platelets, bilirubin and CRP. The error of these parameters are around 7% to 12%. All other parameters estimated using PLS modeling did not met the 15% error criteria for POC (see **Figure 15).** Self-learning AI was able to attain medical analytical grade quality in the following parameters: erythrocytes, hemoglobin, hematocrit, MCV, MCHC, leukocytes, platelets, bilirubin, glucose, myoglobin, CRP, triglycerides and uric acid. Only creatinine and urea quantification were above the 5% limit, but did qualified for POC qualitative analysis. The proposed self-learning artificial intelligence method greatly solves the previous technical barriers presented in the background art, allowing spectroscopy to attain analytical grade errors.

The following pertains to results and discussion, in particular classification. Herein it is also demonstrated the effectiveness of the proposed self-learning method for classification of known health conditions, such as: anemia, leukocytosis, thrombotopenia, thrombocythemia, hepatic insufficiency, diabetes mellitus, acute myocardial infarction, renal dysfunction and inflammation. The classification of these conditions was performed according to the diagnosis cut-off values: i) anemia - erythrocytes count levels below $4*10^{12}$/L and hemoglobin levels below 13 g/dL; ii) leukocytosis - leukocytes levels above $10^{10}$/L; iii) thrombotopenia - platelets levels below $100*10^{9}$/L; iv) thrombocythemia - platelets levels above $400*10^{9}$/L; v) hepatic insufficiency - bilirubin levels above 1.2 mg/dl; vi) diabetes mellitus - glucose levels above 100 mg/dl; vii) acute myocardial infarction - myoglobin levels above 147 ng/ml; viii) renal dysfunction - creatinine levels above 1.3 mg/ml; ix) inflammation - C-reactive protein levels above 2.0 mg/dl.

**Table 2** presents the classification results for the presented conditions, in terms of true and false, positive and negative combinations, respectively. Results show that self-learning classification is superior to a linear classifier, logistic PLS. This is especially significant for conditions where the cut-off value for diagnosis is at low concentrations, such as for thrombotopenia, or for conditions that suffer complex interferences, such as infections with high levels of leukocytes (leukocytosis). The global PLS model is only able to sustain point-of-care (15% error of classification) for anemia, thrombocythemia and acute myocardial infarction. Most parameters exhibit levels of 50% to 80% chance of correct diagnosis, and therefore using linear classifiers proves to be very limited for classification of health conditions.

Self-learning method was able always perform above 85% chances of correct diagnosis. The Self-learning method was able to correctly diagnose 100% of the cases of anemia, thrombocythemia and acute myocardial infarction. Conditions, such as, leukocytosis, diabetes mellitus and hepatic function also attain near complete correct classification (97% chance of being correct). Such, is because, values that are miss classified are near the cut-off, and the laboratory error was not taken into the account in the classification method. If one takes it into consideration, with an error margin of 5%, these conditions are also 100% classified. thrombotopenia and renal dysfunction have classification rate of 87% and 89%, respectively (see Table 2). Such result was expected, as platelets and creatinine values are significantly low for their signal information in the spectra (e.g. creatinine has 14% of prediction using self-learning, see Table 1). Nevertheless, the two conditions are below the 15% classification error.

It will be appreciated by those of ordinary skill in the art that unless otherwise indicated herein, the particular sequence of steps in text or flow diagrams described is illustrative only and can be varied without departing from the disclosure. Thus, unless otherwise stated the steps described are so unordered meaning that, when possible, the steps can be performed in any convenient or desirable order.

It is to be appreciated that certain embodiments of the disclosure as described herein may be incorporated as code (e.g., a software algorithm or program) residing in firmware and/or on computer useable medium having control logic for enabling execution on a computer system having a computer processor, such as any of the servers described herein. Such a computer system typically includes memory storage configured to provide output from execution of the code which configures a processor in accordance with the execution. The code can be arranged as firmware or software, and can be organized as a set of modules, including the various modules and algorithms described herein, such as discrete code modules, function calls, procedure calls or objects in an object-oriented programming environment. If implemented using modules, the code can comprise a single module or a plurality of modules that operate in cooperation with one another to configure the machine in which it is executed to perform the associated functions, as described herein.

The above described embodiments are combinable. The following claims further set out particular embodiments of the disclosure.

References

P. Geladi and B. Kowalsky. Partial least squares regression: a tutorial. Analytical Chemical Acta, 185:1-17, 1986.

A. Phatak and S. Jong. The geometry of partial least squares. Journal of Chemometrics, 11:311-338, 1997.

Huang, G.B. Huan, S. Song, and K. You. Trends in extreme learning machines: A review. Neural Networks, 61:32-48, 2013.

L. Ramirez-Lopez, T. Behrensa, K. Schmidt, A. Stevens, J.A.M. Demattê, and T. Scholten. The spectrum-based learner: A new local approach for modeling soil vis-nir spectra of complex datasets. Geoderma, 195 - 196:268 - 279, 2013.

D.P. Solomatine, Maskey. M., and Shrestha. D.L. Instance-based learning compared to other data-driven methods in hydrological forecasting. Hydrol. Process, 22:275 - 287, 2008.

T. Naes, T. Isaksson, and B. Kowalski. Locally weighted regression and scatter correction for near-infrared reflectance data. Anal. Chem., 62(7):664 - 673, 1990.

C.D. Christy and S.A. Dyer. Estimation of soil properties using a combination of spectral and scalar sensor data.

J.S. Shenk, M.O. Westerhaus, and P.erzaghi. Local prediction with near infrared multi-product databases. Journal of Near Infrared Spectroscopy, 5:223-232, 1997.

T. Fearn and A.M.C. Davies. Locally-biased regression. Journal of Near Infrared, 11(6):467 - 478, 2003.
A.M.C Davies and T.earn. Quantitative analysis via near infrared databases: comparison analysis using restructured nearest infrared and constituent data-deux (carnac-d). Journal of Near Infrared, 14(6):403 - 411, 2003.
F.Goge, R. Joffre, C. Jolivet, I. Ross, and L. Ranjard. Optimization criteria in sample selection step of local regression for quantitative analysis of large soil nirs database. Chemometrics and Intelligent Laboratory Systems, 110(1):168-176, 2012.
L. Ramirez-Lopez, T. Behrens, K. Schmidt, A. Stevens, J.A.M. Demattê, and T. Scholten. The spectrum-based learner: a new local approach for modelling soil vis-nir spectra of complex datasets.
Geoderma, 195-196:268-279, 2013.
L. Ramirez-Lopez, T.Behrens, K.Schmidt, R.A. ViscarraRossel, J.A.M. Demattê, and T. Scholten. Distance and similarity-search metrics for use with soil vis nir spectra. Geoderma, 199:43-53, 2013.
U.G. Indahl. The geometry of pls1 explained properly: 10 key notes on mathematical properties of and some alternative algorithmic approaches to pls1 modelling. Journal of Chemometrics, 24:168-180, 2014.
R.J. Pell, L.S. Ramos, and R. Manne. The model space in partial least squares regression. Journal of Chemometrics, 21:165-172, 2007.
S. Wold, M. Hoyc, H. Martens, J. Trygg, F. Westade, J. MacGregor, and B.M. Wise. The pls model space revisited. Journal of Chemometrics, 23:67-68, 2009.
R. Ergon. Finding y-relevant part of x by use of pcr and plsr model reduction methods. Journal of Chemometrics, 21:537-546, 2007.
R. Ergon. Re-interpretation of nipals results solves plsr inconsistency problem. Journal of Chemometrics, 23:72-75, 2009.
R.C. Martins, V.V. Lopes, P. Valentão, J.C.M.F. Carvalho, P. Isabel, M.T. Amaral, M.T. Batista, P. B. Andrade, and B.M. Silva. Relevant principal component analysis applied to the characterisation of portuguese heather honey. Natural Product Research, 22:1560-1582, 2007.

## Claims

1. Spectrophotometry self-learning computer-implemented method for predicting a quantification of a constituent from a sample to be quantified,
comprising the steps of:

   obtaining an electromagnetic spectrum from said biological sample;
   projecting said obtained spectrum into a sample point of a multiple dimension vector space associated with feature vectors, herewith a feature space, defined by a predetermined vector basis, wherein each of said dimensions is a prediction feature;
   selecting, if existing, a minimum of neighbouring sample points from sample points within said feature space, said sample points having been projected from previously obtained spectra each with a known constituent quantity, such that said minimum maximises the covariance of the projected spectrum of said sample to be quantified together with the projected spectra of the selected neighbouring sample points;
   predicting the quantification of the constituent from the sample to be quantified by correlating the known constituent quantity from the selected neighbouring sample points taking into consideration the projected spectrum of said sample to be quantified and the projected spectra of said selected neighbouring sample points.

2. Method according to the previous claim further comprising for determining the predictability of quantification of the constituent of the sample to be quantified, by:

   calculating a normal distribution of the prediction error of the constituent quantity from the selected neighbouring sample points;
   obtaining a p-value from said calculated normal distribution and from the projected spectrum of said sample to be quantified;
   using the obtained p-value as the predictability of quantification of the constituent of the sample to be quantified.

3. Method according to any of the previous claims further comprising, if the minimum of neighbouring sample points is not existing, the steps of:
flagging that prediction of the quantification of the constituent from the sample to be quantified is not possible.

4. Method according to claim 2 or 3, further comprising:

placing the obtained spectrum from said biological sample into a quarantine database;
receiving a measured quantification of the constituent from the sample to be quantified;
accumulating in said quarantine database a plurality of obtained spectra from biological samples to be quantified and respective measured quantifications of the constituent from samples to be quantified;
determining if a subset of the accumulated spectra and measured quantifications in said quarantine database allows predictability of quantification of the constituent of the sample to be quantified;
if the subset allows predictability, releasing the subset from the quarantine database for use in the present spectrophotometry method for predicting a quantification of a constituent.

**5.** Method according to the previous claim, determining if a subset of the accumulated spectra and measured quantifications allows predictability of quantification of the constituent of the sample to be quantified, comprises:

on placing the obtained spectrum from a biological sample into the quarantine database; and
on receiving a measured quantification of the constituent from the sample to be quantified corresponding to the biological sample;
the steps of:

projecting said obtained spectrum into a sample point of a multiple dimension vector space associated with feature vectors, herewith a feature space, defined by a predetermined vector basis, wherein each of said dimensions is a prediction feature;
selecting, from said quarantine database, if existing, a minimum of neighbouring sample points from sample points within said feature space, said sample points having been projected from previously obtained spectra each with a known constituent quantity, such that said minimum maximises the covariance of the projected spectrum of said sample to be quantified together with the projected spectra of the selected neighbouring sample points of said quarantine database;
predicting the quantification of the constituent from the sample to be quantified by correlating the known constituent quantity from the selected neighbouring sample points from said quarantine database taking into consideration the projected spectrum of said sample to be quantified and the projected spectra of said selected neighbouring sample points of said quarantine database;
determining the predictability of quantification of the constituent of the sample to be quantified;
if the predictability is deemed above a predetermined threshold, releasing the selected neighbouring sample points, said released selected neighbouring sample points constituting a local model, from said quarantine database for use in the present spectrophotometry method for predicting a quantification of a constituent.

**6.** Method according to any of the previous claims, wherein selecting the minimum of neighbouring sample points from sample points within said feature space, comprises the steps of:

projecting an obtained spectrum into a sample point of the multiple dimension vector space, herewith a feature space;
defining a plurality of search directions in said feature space;
defining a plurality of directional search volumes contained within said feature space, each directional search volume being defined as a region of the feature space that includes and originates from said projected spectrum sample point, that extends along a search direction by a predetermined search radius distance from said projected sample point, and that extends from said search direction by a predetermined search width distance;
calculating for each search direction a plurality of corresponding prediction models, wherein each said model is calculated by selecting a dimension subset from the dimensions of the feature space, said model being calculated using the projected sample points within the directional search volume corresponding to the search direction such that covariance is maximised;
selecting the search direction that has the corresponding prediction model that has a maximum predictability of quantification of the constituent to be quantified; using the projected sample points within a selected directional search volume corresponding to the selected search direction as the selected minimum of neighbouring sample points.

**7.** Method according to claim 6, further comprising:
minimizing the selected directional search volume by reducing the predetermined search width distance such that the predictability of quantification of the constituent to be quantified is maximized by the model calculated by the selected dimension subset and calculated using the projected sample points within the directional search volume being minimized.

**8.** Method according to any of the claims 6-7 wherein the prediction model of each search direction is calculated by:

defining a covariance matrix between the feature space and the quantification of the constituent;
minimizing the number of eigenvectors extracted from the covariance matrix that minimizes prediction error;
selecting those eigenvectors that correspond to said minimum;
using a multivariate linear prediction model defined by the selected eigenvectors as the calculated prediction model of each search direction,
wherein the calculated multivariate linear prediction model joined with the projection defined by the predetermined vector basis provides an interpretive correlation between an input spectrum and constituent quantification,
wherein each said directional search volumes is a multidimensional box or cylinder defined by a predetermined distance from a line defined by the respective search direction and the projected spectrum sample point.

**9.** Method according to any of claims 6-8, comprises repeating a selection the minimum of neighbouring sample points from sample points within said feature space, by the steps of:

defining a plurality of search directions in said feature space;
defining a plurality of directional search volumes contained within said feature space, each directional search volume being defined as a region of the feature space that originates from the end of the predetermined search radius distance along the selected search direction of the previously selected directional search volume;
calculating for each said search direction a plurality of corresponding prediction models, wherein each said model is calculated by selecting a dimension subset from the dimensions of the feature space, said model being calculated using the projected sample points within said directional search volume corresponding to the search direction;
selecting said search direction that has the corresponding prediction model that has a maximum predictability of quantification of the constituent to be quantified; using the projected sample points within a selected directional search volume corresponding to said selected search direction as the selected minimum of neighbouring sample points.

**10.** Method according to the previous claim, comprises repeating the steps above until a predetermined criteria is reached in respect of covariance of the projected spectrum of the sample to be quantified together with the projected spectra of the selected neighbouring sample points.

**11.** Method according to any of the claims 9-10, comprising:

repeating the selection the minimum of neighbouring sample points from projected sample points within said feature space, recursively calculating said prediction models;
aggregating said calculated prediction models into an aggregated prediction model, herewith a path model.

**12.** Method according any of the previous claims where the quantification to be predicted is a logistic function of a class to be determined from a constituent or constituents from the sample.

**13.** Method according to any of the previous claims wherein selecting a minimum of neighbouring sample points from sample points within said feature space comprises selecting a minimum number of sample points above a predetermined threshold of covariance.

**14.** Method according to any of the previous claims wherein the sample is biological and the constituent or constituents are biological metabolites, in particular blood metabolites.

**15.** Spectrophotometry device for predicting a quantification of a constituent from a sample to be quantified, the device comprising an electronic data processor configured for carrying out the spectrophotometry self-learning computer-implemented method of any of the claims 1-14, further comprising a spectrophotometer and non-transitory storage medium including program instructions which, when executed by said electronic data processor, cause it to carry out said spectrophotometry method.

## Patentansprüche

**1.** Selbstlernendes computerimplementiertes spektrophotometrisches Verfahren zur Vorhersage einer Quantifizierung eines Bestandteils aus einer zu quantifizierenden Stichprobe, umfassend die folgenden Schritte:

Gewinnen eines elektromagnetischen Spektrums aus der genannten biologischen Stichprobe;
Projizieren des genannten erhaltenen Spektrums in einen Stichprobenpunkt eines mehrdimensionalen Vektorraums, der mit Merkmalsvektoren, und damit einem Merkmalsraum, assoziiert ist, der durch eine vorbestimmte Vektorbasis definiert ist, wobei jede der genannten Dimensionen ein Vorhersagemerkmal ist;
Auswählen, falls vorhanden, eines Minimums benachbarter Stichprobenpunkte aus den Stichprobenpunkten innerhalb des genannten Merkmalsraums, wobei die genannten Stichprobenpunkte aus zuvor erhaltenen Spektren projiziert wurden, von denen jedes eine bekannte konstituierende Größe aufweist, so dass das genannte Minimum die Kovarianz des projizierten Spektrums der genannten zu quantifizierenden Stichprobe zusammen mit den projizierten Spektren der ausgewählten benachbarten Stichprobenpunkte maximiert;
Vorhersagen der Quantifizierung des Bestandteils aus der zu quantifizierenden Stichprobe durch Korrelation der bekannten Bestandteilsmenge aus den ausgewählten benachbarten Stichprobenpunkten unter Berücksichtigung des projizierten Spektrums der genannten zu quantifizierenden Stichprobe und der projizierten Spektren der genannten ausgewählten benachbarten Stichprobenpunkte.

2. Verfahren nach dem vorangehenden Anspruch, ferner umfassend das Bestimmen der Vorhersagbarkeit der Quantifizierung des Bestandteils der zu quantifizierenden Stichprobe, durch:

Berechnen einer Normalverteilung des Vorhersagefehlers der konstituierenden Größe aus den ausgewählten benachbarten Stichprobenpunkten;
Ermitteln eines p-Wertes aus der genannten berechneten Normalverteilung und dem projizierten Spektrum der genannten zu quantifizierenden Stichprobe;
Verwenden des erhaltenen p-Wertes als die Vorhersagbarkeit der Quantifizierung des Bestandteils der zu quantifizierenden Stichprobe.

3. Verfahren nach einem der vorangehenden Ansprüche, ferner umfassend, wenn das Minimum an benachbarten Stichprobenpunkt nicht vorhanden ist, die folgenden Schritte: Anzeigen, dass eine Vorhersage der Quantifizierung des Bestandteils aus der zu quantifizierenden Stichprobe nicht möglich ist.

4. Verfahren nach Ansprüchen 3 oder 2, ferner umfassend:

Aufnehmen des erhaltenen Spektrums der genannten biologischen Stichprobe in eine Quarantänedatenbank;
Empfangen einer gemessenen Quantifizierung des Bestandteils aus der zu quantifizierenden Stichprobe;
Sammeln einer Vielzahl erhaltener Spektren aus zu quantifizierenden biologischen Stichproben und entsprechenden gemessenen Quantifizierungen des Bestandteils der zu quantifizierenden Stichproben in der genannten Quarantänedatenbank;
Bestimmen, ob eine Teilmenge der gesammelten Spektren und gemessenen Quantifizierungen in der genannten Quarantänedatenbank eine Vorhersagbarkeit der Quantifizierung des Bestandteils der zu quantifizierenden Stichprobe ermöglicht;
wenn die Teilmenge eine Vorhersage ermöglicht, Freigeben der Teilmenge aus der Quarantänedatenbank zur Verwendung in dem vorliegenden spektrophotometrischen Verfahren zur Vorhersage einer Quantifizierung eines Bestandteils.

5. Verfahren nach dem vorangehenden Anspruch, wobei bestimmt wird, ob eine Teilmenge der gesammelten Spektren und gemessenen Quantifizierungen eine Vorhersagbarkeit der Quantifizierung des Bestandteils der zu quantifizierenden Stichprobe ermöglicht, umfassend:

beim Ablegen des erhaltenen Spektrums einer biologischen Probe in der Quarantänedatenbank; und
beim Empfangen einer gemessenen Quantifizierung der Komponente aus der zu quantifizierenden Probe, die der biologischen Probe entspricht;
die folgenden Schritte:

Projizieren des genannten erhaltenen Spektrums in einen Stichprobenpunkt eines mehrdimensionalen Vektorraums, der mit Merkmalsvektoren, und damit einem Merkmalsraum, assoziiert ist, der durch eine vorbestimmte Vektorbasis definiert ist, wobei jede der genannten Dimensionen ein Vorhersagemerkmal ist;
Auswählen aus der genannten Quarantänedatenbank, falls vorhanden, eines Minimums benachbarter Stichprobenpunkte aus den Stichprobenpunkten innerhalb des genannten Merkmalsraums, wobei die genannten Stichprobenpunkte aus zuvor erhaltenen Spektren projiziert wurden, von denen jedes eine bekannte konstituierende Größe aufweist, so dass das genannte Minimum die Kovarianz des projizierten Spektrums der genannten zu quantifizierenden Stichprobe zusammen mit den projizierten Spektren der ausgewählten benachbarten Stichprobenpunkte aus der genannten Quarantänedatenbank maximiert;
Vorhersagen der Quantifizierung des Bestandteils aus der zu quantifizierenden Stichprobe durch Korrelation der bekannten Bestandteilsmenge aus den ausgewählten benachbarten Stichprobenpunkten aus der genannten Quarantänedatenbank unter Berücksichtigung des projizierten Spektrums der genannten zu quantifizierenden Stichprobe und der projizierten Spektren der genannten ausgewählten benachbarten Stichprobenpunkte aus der genannten Quarantänedatenbank.
Bestimmen der Vorhersagbarkeit der Quantifizierung des Bestandteils der zu quantifizierenden Stichprobe;
falls die Vorhersagbarkeit als oberhalb eines vorbestimmten Schwellenwerts erachtet wird, Freigeben der ausgewählten benachbarten Stichprobenpunkte, wobei diese zusammen mit den genannten freigegebenen ausgewählten benachbarten Stichprobenpunkten ein lokales Modell aus der Quarantäne-Datenbank, das in dem vorliegenden spektrophotometrischen Verfahren zur Vorhersage der Quantifizierung einer Komponente verwendet wird, bilden.

**6.** Verfahren nach einem der vorangehenden Ansprüche, wobei das Auswählen des Minimums benachbarter Stichprobenpunkt aus Stichprobenpunkten innerhalb des genannten Merkmalsraums die folgenden Schritte umfasst:

Projizieren eines erhaltenen Spektrums in einen Stichprobenpunkt des mehrdimensionalen Vektorraums, hier eines Merkmalsraums;
Definieren einer Vielzahl von Suchrichtungen in dem genannten Merkmalsraum;
Definieren einer Vielzahl gerichteter Suchvolumina innerhalb des genannten Merkmalsraum, wobei jedes gerichtete Suchvolumen als ein Bereich des Merkmalsraums definiert ist, der den genannten projizierten spektralen Stichprobenpunkt einschließt und von diesem ausgeht, der sich entlang einer Suchrichtung um einen vorbestimmten Suchradiusabstand von dem genannten projizierten Stichprobenpunkt erstreckt und sich von der genannten Suchrichtung um einen vorbestimmten Suchbreitenabstand erstreckt; Berechnen einer Vielzahl entsprechender Vorhersagemodelle für jede Suchrichtung,
wobei jedes der genannten Modelle durch Auswählen einer Dimensionsteilmenge aus den Dimensionen des Merkmalsraums berechnet wird, wobei das genannte Modell unter Verwendung der projizierten Stichprobenpunkte innerhalb des der Suchrichtung entsprechenden Richtungssuchvolumens berechnet wird, so dass die Kovarianz maximiert wird;
Auswählen der Suchrichtung mit dem entsprechenden Vorhersagemodell, das eine maximale Vorhersagbarkeit der Quantifizierung des zu quantifizierenden Bestandteils aufweist;
Verwenden der projizierten Stichprobenpunkte innerhalb eines ausgewählten gerichteten Suchvolumens, das der ausgewählten Suchrichtung entspricht, als ausgewähltes Minimum der benachbarten Stichprobenpunkte.

**7.** Verfahren nach Anspruch 6, ferner umfassend:
Minimieren des ausgewählten gerichteten Suchvolumens durch Verringern des vorbestimmten Suchbreiten-Abstands, so dass die Vorhersagbarkeit der Quantifizierung des zu quantifizierenden Bestandteils durch das Modell maximiert wird, das durch die ausgewählte Dimensionsteilmenge berechnet wird und unter Verwendung der innerhalb des minimierten gerichteten Suchvolumens projizierten Abtastpunkte berechnet wird.

**8.** Verfahren nach einem der Ansprüche 6-7, wobei das Vorhersagemodell für jede Suchrichtung berechnet wird durch:

Definieren einer Kovarianzmatrix zwischen dem Merkmalsraum und der Quantifizierung der Konstituente;
Minimieren der Anzahl der Eigenvektoren, die aus der Kovarianzmatrix extrahiert werden und den Vorhersagefehler minimieren;
Auswählen derjenigen Eigenvektoren, die dem genannten Minimum entsprechen;
Verwenden eines multivariaten linearen Vorhersagemodells, das durch die ausgewählten Eigenvektoren als das berechnete Vorhersagemodell jeder Suchrichtung definiert ist,
wobei das berechnete multivariate lineare Vorhersagemodell zusammen mit der durch die vorbestimmte Vektorbasis definierte Projektion, eine interpretative Korrelation zwischen einem Eingangsspektrum und der Quantifizierung der Bestandteile liefert,
wobei jedes der genannten gerichteten Suchvolumina ein mehrdimensionaler Kasten oder Zylinder ist, der durch einen vorbestimmten Abstand von einer Linie definiert ist, die durch die jeweilige Suchrichtung und den projizierten spektralen Stichprobenpunkt definiert ist.

**9.** Verfahren nach einem der Ansprüche 6-8, umfassend die Wiederholung einer Auswahl des Minimums benachbarter Stichprobenpunkte aus Stichprobenpunkt innerhalb des genannten Merkmalsraums durch die folgenden Schritte:

Definieren einer Vielzahl von Suchrichtungen in dem genannten Merkmalsraum;
Definieren einer Vielzahl gerichteter Suchvolumina innerhalb des genannten Merkmalsraum, wobei jedes gerichtete Suchvolumen als ein Bereich des Merkmalsraums definiert ist, der von dem Ende des vorbestimmten Suchradiusabstandes entlang der ausgewählten Suchrichtung des zuvor ausgewählten gerichteten Suchvolumens ausgeht;
Berechnen einer Vielzahl entsprechender Vorhersagemodelle für jede genannte Suchrichtung, wobei jedes der genannten Modelle durch Auswählen einer Dimensionsteilmenge aus den Dimensionen des Merkmalsraums berechnet wird, wobei das genannte Modell unter Verwendung der projizierten Stichprobenpunkte innerhalb des der genannten Suchrichtung entsprechenden Richtungssuchvolumens berechnet wird;
Auswählen der genannten Suchrichtung mit dem entsprechenden Vorhersagemodell, das eine maximale Vorhersagbarkeit der Quantifizierung des zu quantifizierenden Bestandteils aufweist;
Verwenden der projizierten Stichprobenpunkte innerhalb eines ausgewählten gerichteten Suchvolumens, das der genannten ausgewählten Suchrichtung entspricht, als ausgewähltes Minimum der benachbarten Stichprobenpunkte.

**10.** Verfahren nach dem vorangehenden Anspruch, umfassend das Wiederholen der obigen Schritte, bis ein vorbestimmtes Kriterium in Bezug auf die Kovarianz des projizierten Spektrums der zu quantifizierenden Stichprobe zusammen mit den projizierten Spektren der ausgewählten benachbarten Stichprobenpunkte erreicht ist.

**11.** Verfahren nach einem der Ansprüchen 9-10, umfassend:

Wiederholen der Auswahl des Minimums benachbarter Stichprobenpunkte aus projizierten Stichprobenpunkten innerhalb des genannten Merkmalsraums und rekursives Berechnen der genannten Vorhersagemodelle;
Aggregieren der genannten berechneten Vorhersagemodelle zu einem aggregierten Vorhersagemodell, hier einem Pfadmodell.

**12.** Verfahren nach einem der vorangehenden Ansprüche, wobei die vorherzusagende Quantifizierung eine logistische Funktion einer aus einem oder mehreren Bestandteilen der Stichprobe zu bestimmenden Klasse ist.

**13.** Verfahren nach einem der vorangehenden Ansprüche, wobei das Auswählen des Minimums benachbarter Stichprobenpunkte aus Stichprobenpunkten innerhalb des genannten Merkmalsraums das Auswählen einer Mindestanzahl von Stichprobenpunkten oberhalb eines vorbestimmten Kovarianz-Schwellenwerts umfasst.

**14.** Verfahren nach einem der vorangehenden Ansprüche, wobei die Stichprobe biologisch ist und der Bestandteil oder die Bestandteile biologische Metaboliten, insbesondere Blutmetaboliten, sind.

**15.** Spektrophotometrische Vorrichtung zur Vorhersage einer Quantifizierung eines Bestandteils aus einer zu quantifizierenden Stichprobe, wobei die Vorrichtung einen elektronischen Datenprozessor umfasst, der so konfiguriert ist, dass er das selbstlernende computerimplementierte spektrophotometrische Verfahren nach einem der Ansprüche 1-14 ausführt, und ferner ein Spektrophotometer und ein nichtflüchtiges Speichermedium umfasst, das Programmanweisungen enthält, die, wenn sie von dem elektronischen Datenprozessor ausgeführt werden, diesen veranlassen, das genannte spektrophotometrische Verfahren auszuführen.

## Revendications

**1.** Procédé de spectrophotométrie d'auto-apprentissage mis en œuvre par ordinateur destiné à prédire une quantification d'un constituant à partir d'un échantillon devant être quantifié, comprenant les étapes consistant à :

obtenir un spectre électromagnétique à partir dudit échantillon biologique ;
projeter ledit spectre obtenu sur un point échantillon d'un espace vectoriel à plusieurs dimensions associé à des vecteurs de caractéristique, en l'occurrence un espace caractéristique, défini par une base vectorielle prédéterminée, dans lequel chacune desdites dimensions est une caractéristique de prédiction ;
sélectionner, si existant, un minimum de points échantillon voisins des points échantillon au sein dudit espace caractéristique, lesdits points échantillon ayant été projetés à partir de spectres préalablement obtenus chacun avec une quantité de constituants connue, tel que ledit minimum maximise la covariance du spectre projeté dudit échantillon devant être quantifié avec les spectres projetés des points échantillon voisins sélectionnés ;
prédire la quantification du constituant à partir de l'échantillon devant être quantifié en corrélant la quantité de constituant connue à partir des points échantillon voisins sélectionnés tenant compte du spectre projeté dudit échantillon devant être quantifié et des spectres projetés desdits points échantillon voisins sélectionnés.

**2.** Procédé selon la revendication précédente comprenant également déterminer la prévisibilité de quantification du constituant de l'échantillon devant être quantifié, en :

calculant une distribution normale de l'erreur de prédiction de la quantité de constituants à partir des points échantillon voisins sélectionnés ;
obtenant une valeur p à partir de ladite distribution normale calculée et à partir du spectre projeté dudit échantillon devant être quantifié ;
utilisant la valeur p obtenue en tant que prévisibilité de quantification du constituant de l'échantillon devant être quantifié.

**3.** Procédé selon l'une quelconque des revendications précédentes comprenant également, si le minimum de points échantillon voisins n'existe pas, les étapes consistant à :
signaliser que la prédiction de la quantification du constituant à partir de l'échantillon devant être quantifié est impossible.

**4.** Procédé selon la revendication 2 ou 3 comprenant également :

placer le spectre obtenu à partir dudit échantillon biologique dans une base de données de quarantaine ;
recevoir une quantification mesurée du constituant à partir de l'échantillon devant être quantifié ;
accumuler dans ladite base de données de quarantaine une pluralité de spectres obtenus à partir d'échantillons biologiques devant être quantifiés et les quantifications mesurées respectives des constituants à partir d'échantillons devant être quantifiés ;
déterminer si un sous-ensemble des spectres accumulés et des quantifications mesurées dans ladite base de données de quarantaine permet une prévisibilité de quantification du constituant de l'échantillon devant être quantifié ;
si le sous-ensemble permet la prévisibilité, retirer le sous-ensemble de la base de données de quarantaine pour une utilisation dans le procédé de spectrophotométrie présent destiné à prédire une quantification d'un constituant.

**5.** Procédé selon la revendication précédente dans lequel déterminer si un sous-ensemble des spectres accumulés et des quantifications mesurées permet la prévisibilité de quantification du constituant de l'échantillon devant être quantifié, comprend :

en plaçant le spectre obtenu à partir d'un échantillon biologique dans la base de données de quarantaine ; et
en recevant une quantification mesurée du constituant à partir de l'échantillon devant être quantifié correspondant à l'échantillon biologique ;
les étapes consistant à :

projeter ledit spectre obtenu sur un point échantillon d'un espace vectoriel à plusieurs dimensions associé à des vecteurs de caractéristique, en l'occurrence un espace caractéristique, défini par une base vectorielle prédéterminée, dans lequel chacune desdites dimensions est une caractéristique de prédiction ;
sélectionner, parmi ladite base de données de quarantaine, si existant, un minimum de points échantillon voisins à partir des points échantillon au sein dudit espace de caractéristique, lesdits points échantillon ayant été projetés à partir de spectres préalablement obtenus chacun avec une quantité de constituant connue, tel que ledit minimum maximise la covariance du spectre projeté dudit échantillon devant être quantifié avec les spectres projetés des points échantillon voisins sélectionnés de ladite base de donnés de quarantaine ;
prédire la quantification du constituant à partir de l'échantillon devant être quantifié en corrélant la quantité de constituant connue à partir des points échantillon voisins sélectionnés à partir de ladite base de données de quarantaine tenant compte du spectre projeté dudit échantillon devant être quantifié et des spectres projetés desdits points échantillon voisins sélectionnés de ladite base de données de quarantaine ;
déterminer la prévisibilité de quantification du constituant de l'échantillon devant être quantifié ;
si la prévisibilité est considérée supérieure à un seuil prédéterminé, retirer les points échantillon voisins sélectionnés, lesdits points échantillon voisins sélectionnés retirés constituant un modèle local, de ladite base de données de quarantaine pour une utilisation dans le procédé de spectrophotométrie présent destiné à prédire une quantification d'un constituant.

**6.** Procédé selon l'une quelconque des revendications précédentes, dans lequel sélectionner le minimum de points échantillon voisins à partir des points échantillon au sein dudit espace caractéristique, comprend les étapes consistant à :

projeter un spectre obtenu sur un point échantillon de l'espace vectoriel à plusieurs dimensions, en l'occurrence un espace caractéristique ;
définir une pluralité de directions de recherche dans ledit espace caractéristique ;
définir une pluralité de volumes de recherche directionnels contenus au sein dudit espace caractéristique, chaque volume de recherche directionnel étant défini comme une région de l'espace caractéristique qui inclut et a pour origine ledit point échantillon de spectre projeté, qui s'étend le long d'une direction de recherche par une distance de rayon de recherche prédéterminée à partir dudit point échantillon projeté, et qui s'étend à partir de ladite direction de recherche par une distance de largeur de recherche prédéterminée ;
calculer pour chaque direction de recherche une pluralité de modèles de prédiction correspondants, dans lequel chacun desdits modèles est calculé en choisissant un sous-ensemble de dimension parmi les dimensions de l'espace caractéristique, ledit modèle étant calculé à l'aide des points échantillon projetés au sein du volume de recherche directionnel correspondant à la direction de recherche tel que la covariance soit maximisée ;
sélectionner la direction de recherche qui a le modèle de prédiction correspondant qui a une prévisibilité maximale de quantification du constituant devant être quantifié ;
utiliser les points échantillon projetés au sein d'un volume de recherche directionnel sélectionné correspondant à la direction de recherche sélectionnée en tant que minimum sélectionné des points échantillon voisins.

**7.** Procédé selon la revendication 6 comprenant également :
minimiser le volume de recherche directionnel sélectionné en réduisant la distance de largeur de recherche prédéterminée tel que la prévisibilité de quantification du constituant devant être quantifié soit maximisée par le modèle calculé par le sous-ensemble de dimension sélectionné et calculé à l'aide des points échantillon projetés au sein du volume de recherche directionnel étant minimisé.

**8.** Procédé selon l'une quelconque des revendications 6-7 dans lequel le modèle de prédiction de chaque direction de recherche est calculé en :

définissant une matrice de covariance entre l'espace caractéristique et la quantification du constituant ;
minimisant le nombre de vecteurs propres extraits de la matrice de covariance qui minimise les erreurs de prédiction ;
sélectionnant ces vecteurs propres qui correspondent audit minimum ;
utilisant un modèle de prédiction linéaire multivariable définit par les vecteurs propres sélectionnés en tant que modèle de prédiction calculé de chaque direction de recherche, dans lequel le modèle de prédiction linéaire multivariable calculé joint à la projection définie par la base vectorielle prédéterminée offre une corrélation interprétative entre un spectre d'entrée et la quantification de constituant,
dans lequel chacun desdits volumes de recherche directionnels est une boîte ou un cylindre multidimensionnel défini par une distance prédéterminée à partir d'une ligne définie par la direction de recherche respective et le point échantillon de spectre projeté.

**9.** Procédé selon l'une quelconque des revendications 6-8, qui comprend répéter la sélection du minimum de points échantillon voisins à partir des points échantillon au sein dudit espace caractéristique, à travers les étapes consistant à :

définir une pluralité de directions de recherche dans ledit espace caractéristique ;
définir une pluralité de volumes de recherche directionnels contenus au sein dudit espace caractéristique, chaque volume de recherche directionnel étant défini comme une région de l'espace caractéristique qui a pour origine l'extrémité de la distance de rayon de recherche prédéterminée le long de la direction de recherche sélectionnée du volume de recherche directionnel préalablement sélectionné ;
calculer pour chacune desdites directions de recherche une pluralité de modèles de prédiction correspondants, dans lequel chacun desdits modèles est calculé en sélectionnant un sous-ensemble de dimension parmi les dimensions de l'espace caractéristique, ledit modèle étant calculé à l'aide des points échantillon projetés au sein dudit volume de recherche directionnel correspondant à la direction de recherche ;
sélectionner ladite direction de recherche qui a le modèle de prédiction correspondant qui a une prévisibilité maximale de quantification du constituant devant être quantifiée ;
utiliser les points échantillon projetés au sein d'un volume de recherche directionnel sélectionné correspondant à ladite direction de recherche sélectionnée en tant que minimum sélectionné des points échantillon voisins.

**10.** Procédé selon la revendication précédente, comprenant répéter les étapes ci-dessus jusqu'à ce qu'un critère prédéterminé soit atteint concernant la covariance du spectre projeté de l'échantillon devant être quantifié avec les spectres projetés des points échantillon voisins sélectionnés.

**11.** Procédé selon l'une quelconque des revendications 9-10, comprenant :

répéter la sélection du minimum de points échantillon voisins à partir des points échantillon projetés au sein dudit espace caractéristique, calculant de manière récursive lesdits modèles de prédiction ;
agréger lesdits modèles de prédiction calculés en un modèle de prédiction agrégé, en l'occurrence un modèle de parcours.

**12.** Procédé selon l'une quelconque des revendications précédentes dans lequel la quantification devant être prédite est une fonction logistique d'une classe devant être déterminée à partir d'un constituant ou de constituants de l'échantillon.

**13.** Procédé selon l'une quelconque des revendications précédentes dans lequel sélectionner un minimum de points échantillon voisins à partir des points échantillon au sein dudit espace caractéristique comprend sélectionner un nombre minimum de points échantillon au-dessus d'un seuil prédéterminé de covariance.

**14.** Procédé selon l'une quelconque des revendications précédentes dans lequel l'échantillon est biologique et le constituant ou les constituants sont des métabolites biologiques, en particulier des métabolites sanguins.

**15.** Dispositif de spectrophotométrie destiné à prédire une quantification d'un constituant à partir d'un échantillon devant être quantifié, le dispositif comprenant un traiteur de données électronique configuré pour exécuter le procédé de spectrophotométrie d'auto-apprentissage mis en œuvre par ordinateur de l'une quelconque des revendications 1-14, comprenant également un spectrophotomètre et un moyen de stockage non transitoire incluant des instructions de logiciel qui, lorsqu'il est exécuté par ledit traiteur de données électronique, fait en sorte qu'il exécute ledit procédé de spectrophotométrie.

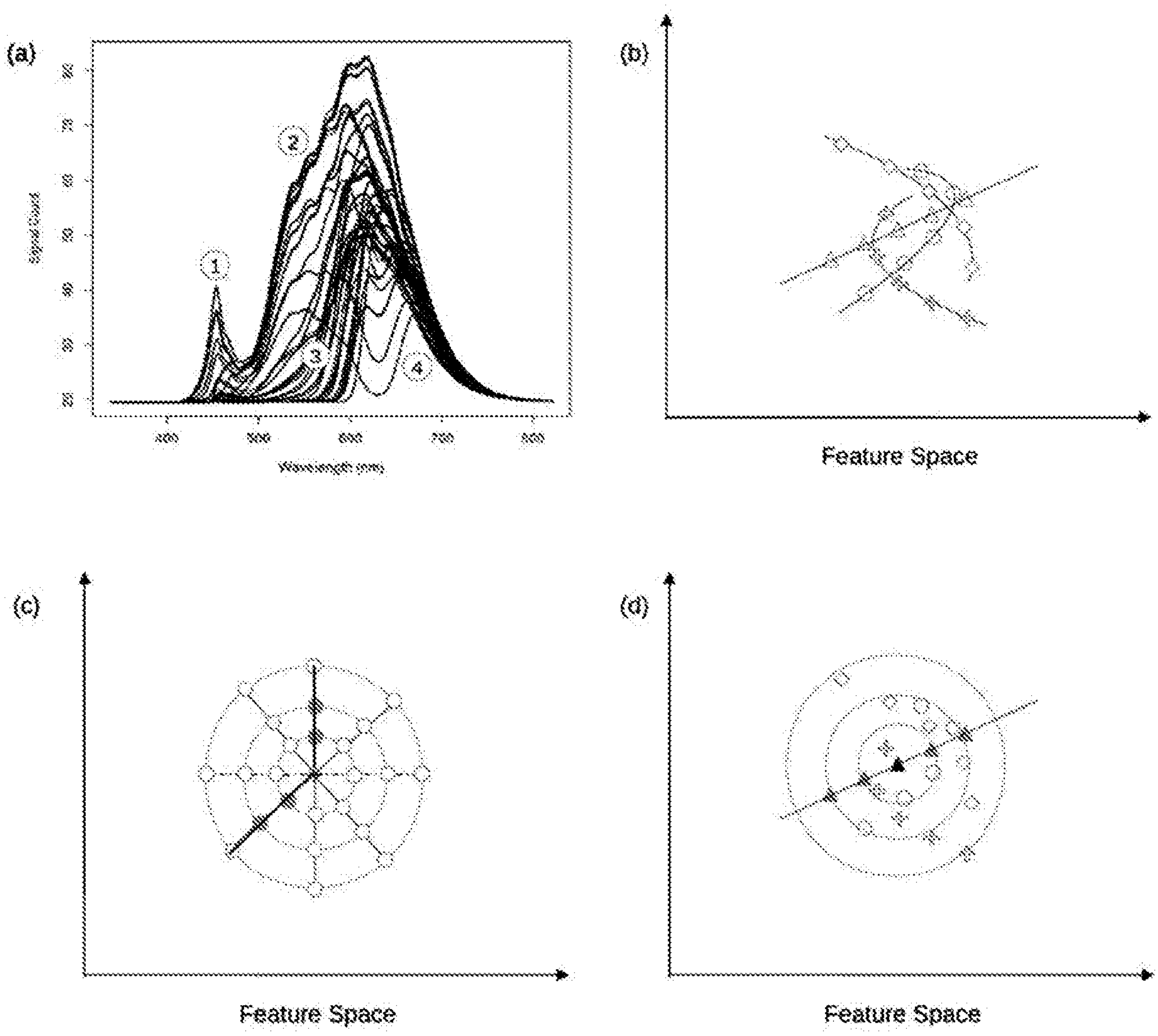
(a)
Signal Count
Wavelength (nm)
1
2
3
4
(b)
Feature Space
(c)
Feature Space
(d)
Feature Space

FIG. 1

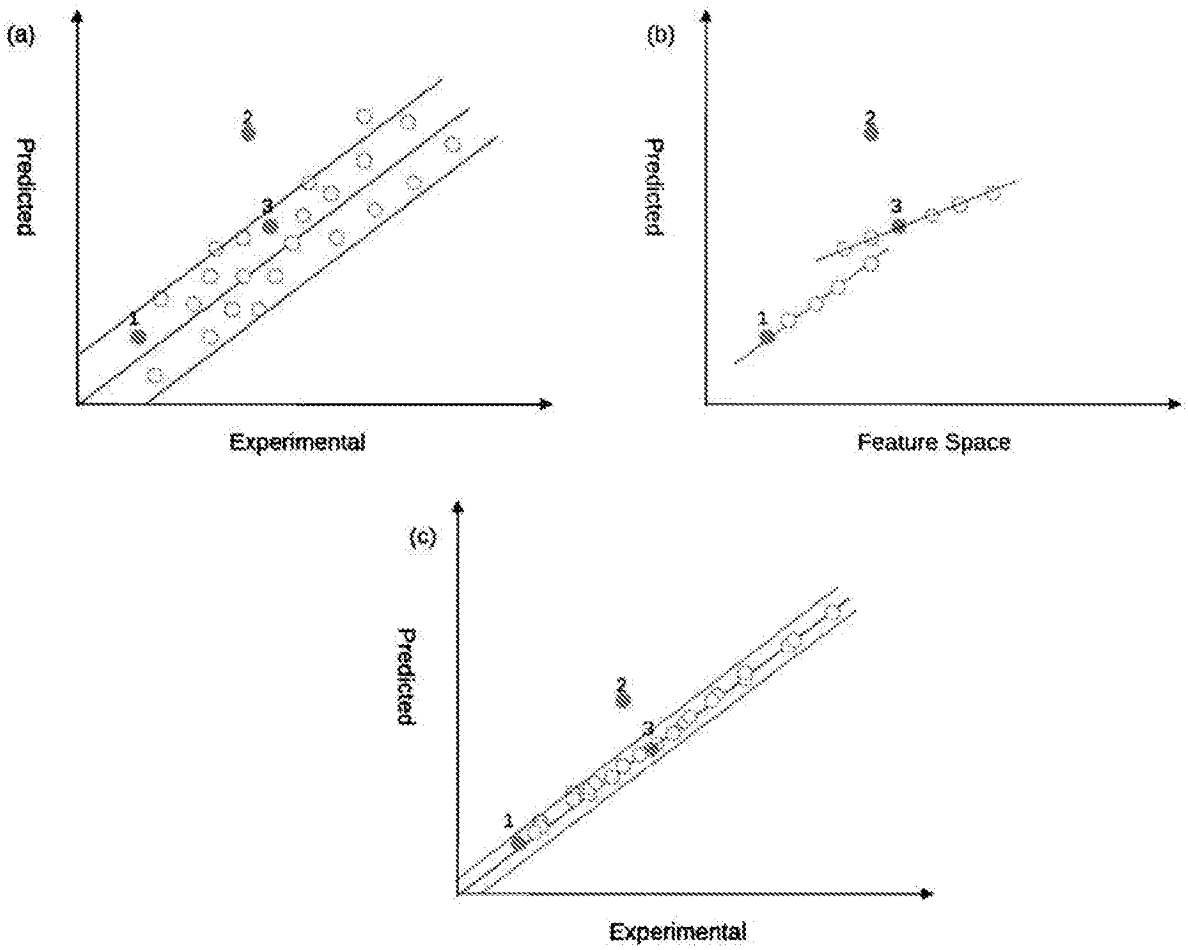
(a)
Predicted
Experimental
2
3
1
(b)
Predicted
Feature Space
2
3
1
(c)
Predicted
Experimental
2
3
1

FIG. 2

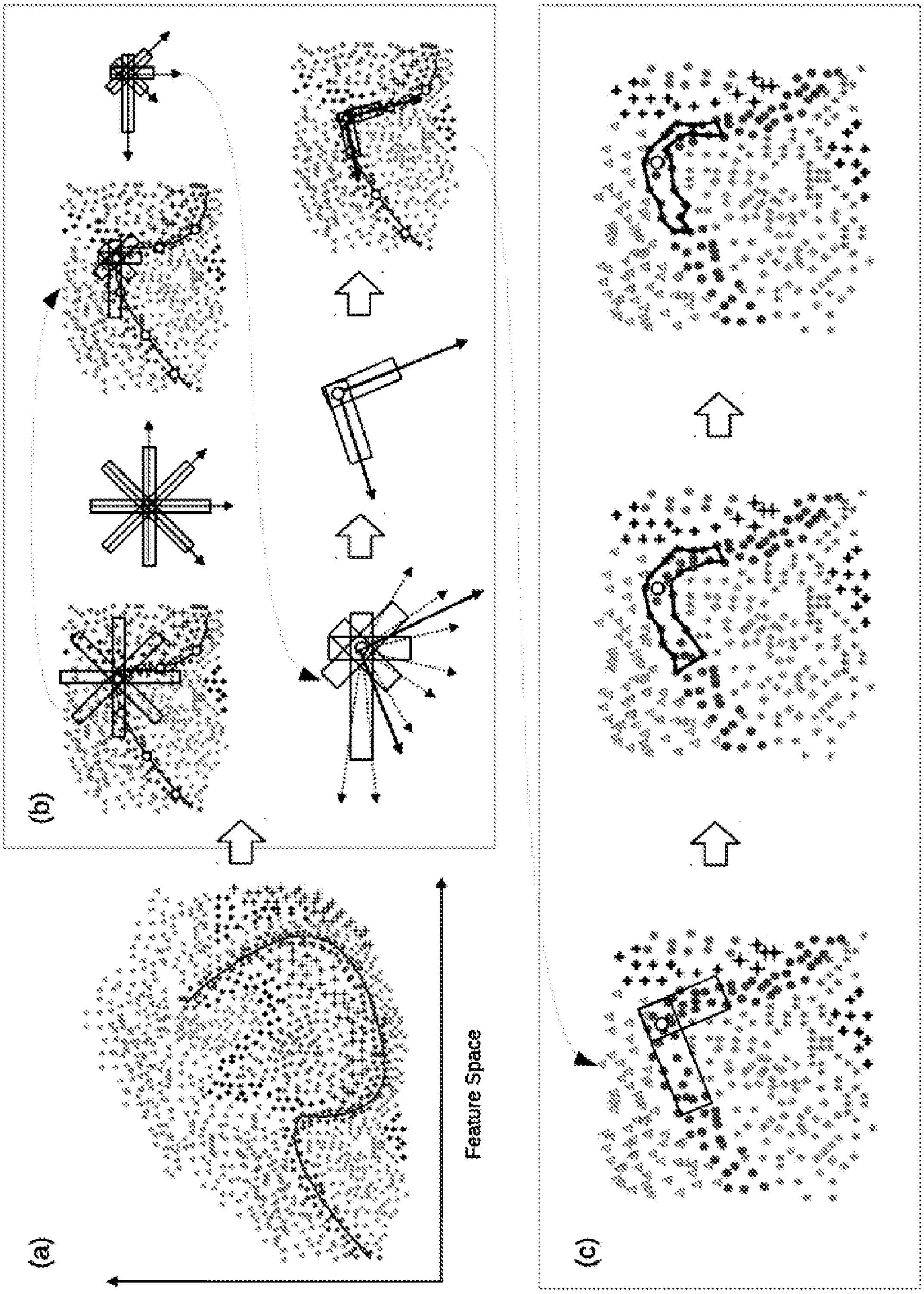


FIG. 3

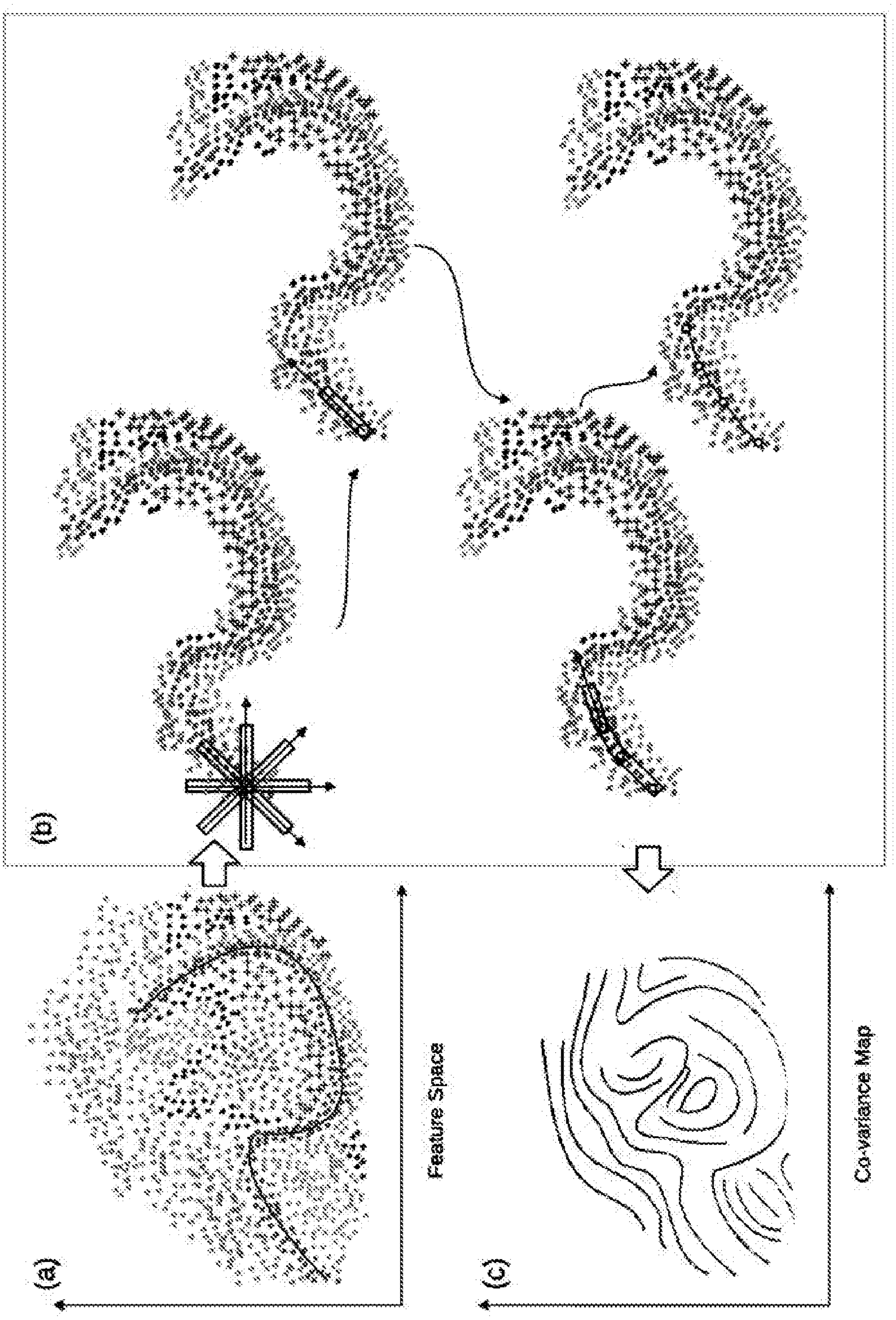


FIG. 4

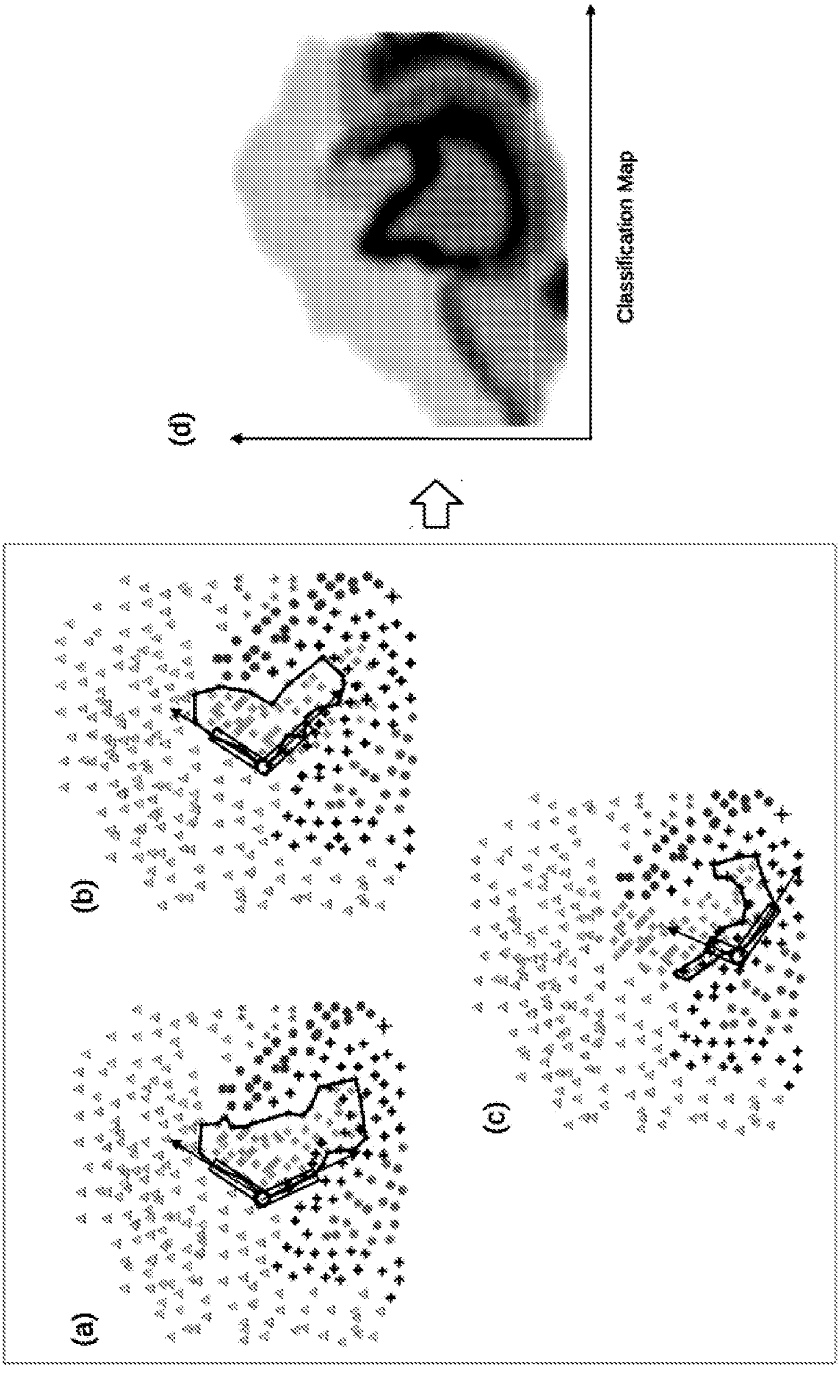


FIG. 5

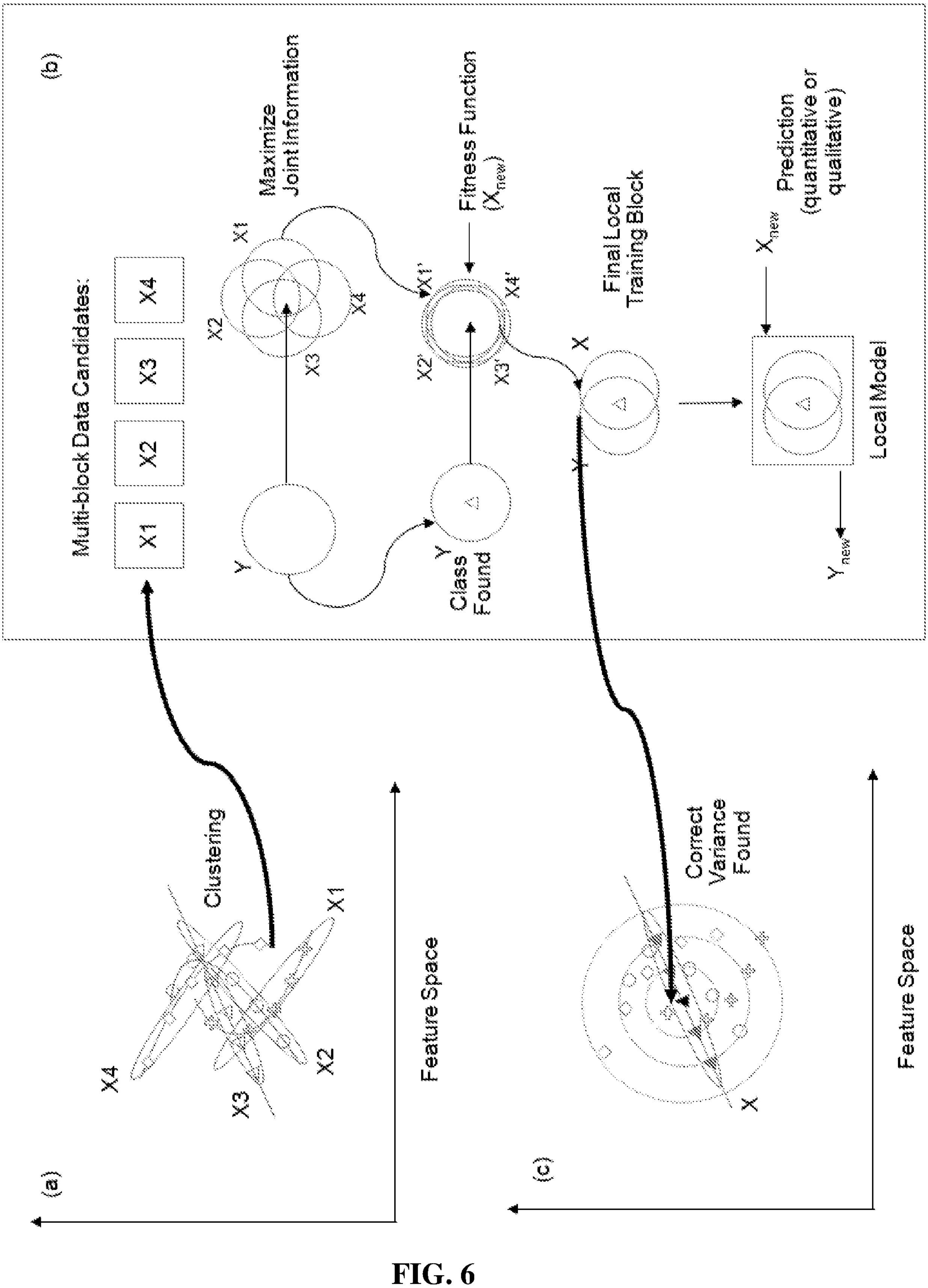
(a)
Clustering
X1
X2
X3
X4
Feature Space
(b)
Multi-block Data Candidates:
X1
X2
X3
X4
Maximize Joint Information
Y
Fitness Function ($X_{new}$)
X1'
X2'
X3'
X4'
Y Class Found
Final Local Training Block
X
$X_{new}$
Prediction (quantitative or qualitative)
Local Model
$Y_{new}$
(c)
Correct Variance Found
X
Feature Space

FIG. 6

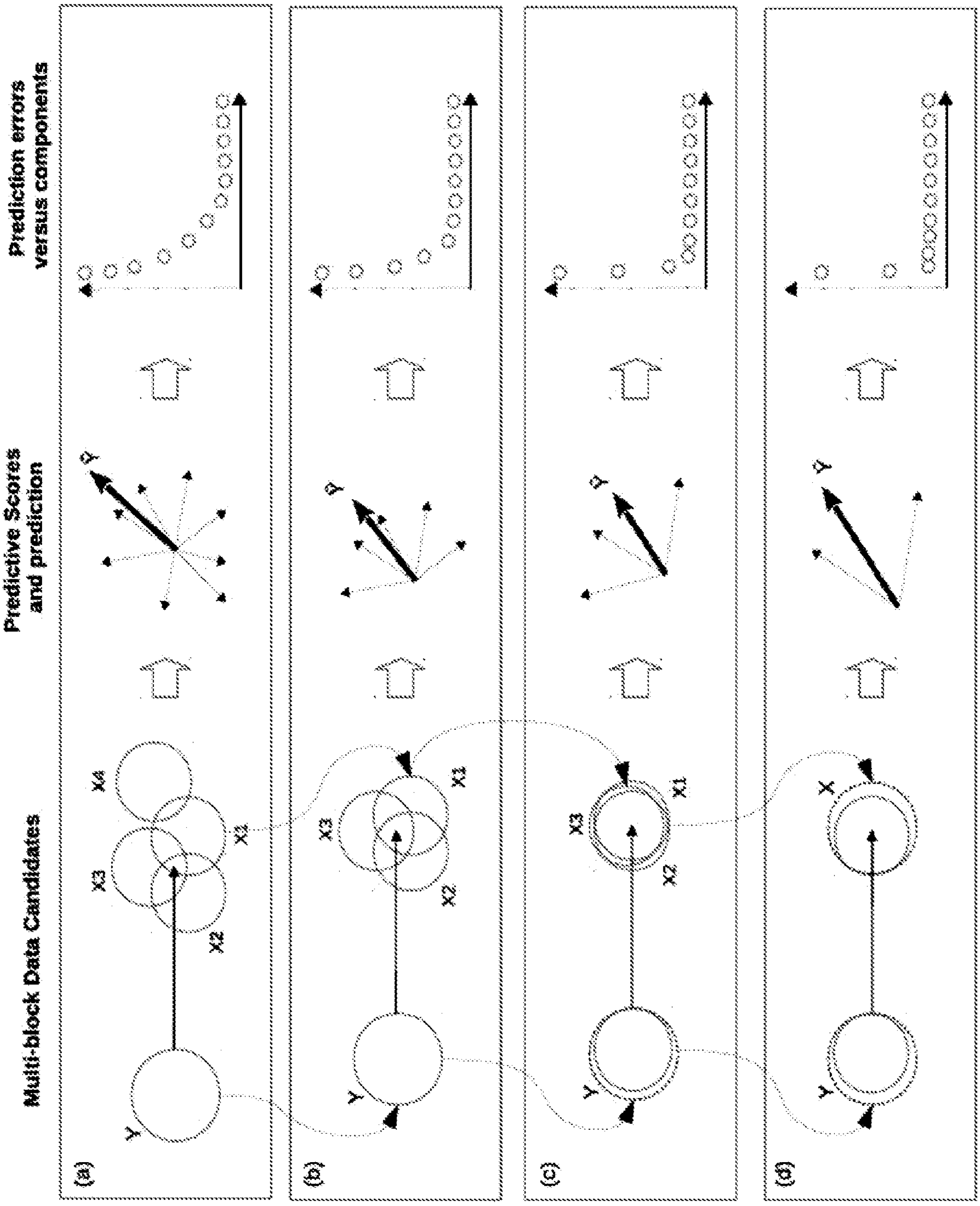
Multi-block Data Candidates
Predictive Scores and prediction
Prediction errors versus components
(a)
Y
X1
X2
X3
X4
(b)
Y
X1
X2
X3
(c)
Y
X1
X2
X3
(d)
Y
X

FIG. 7

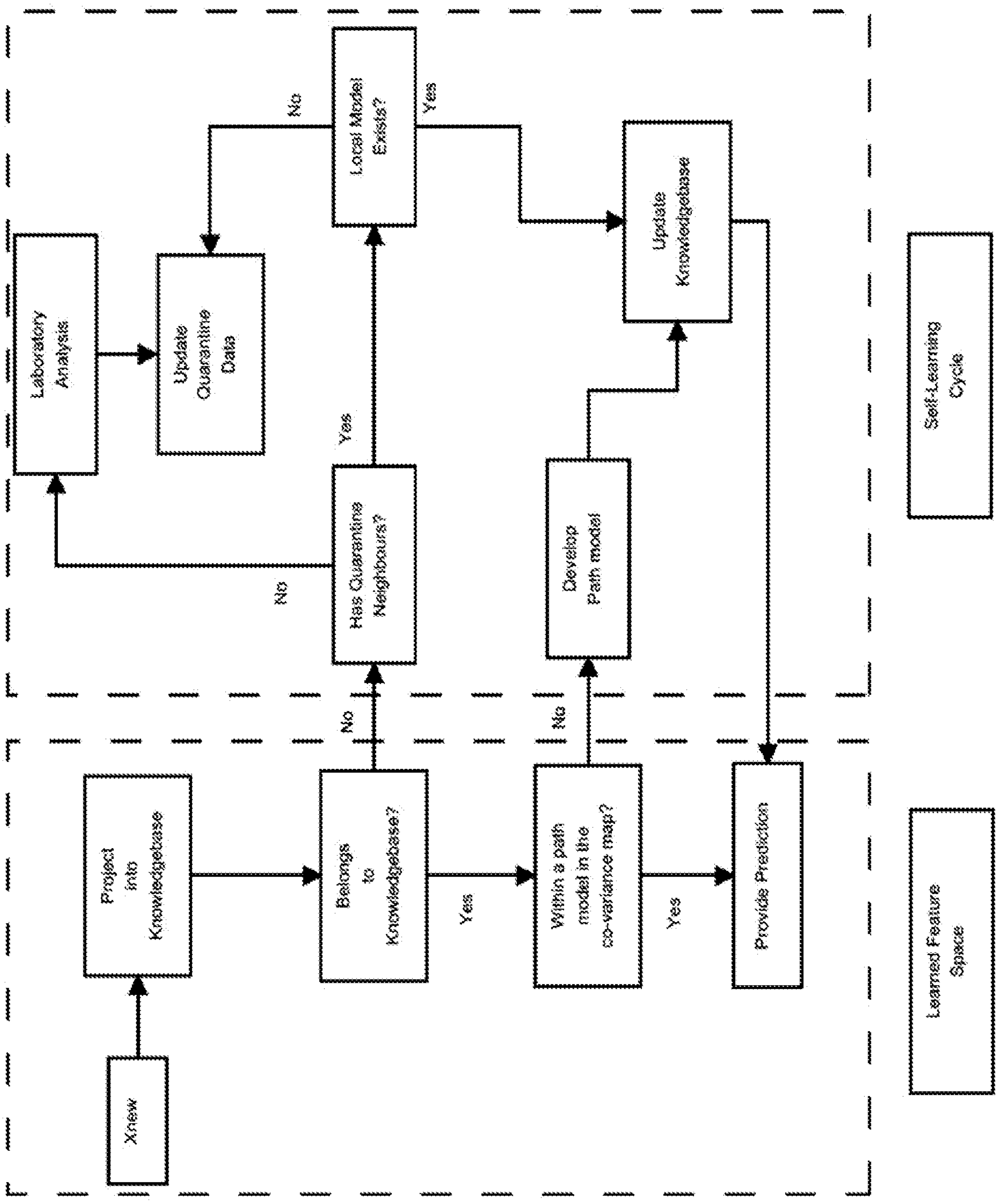
Xnew
Project into Knowledgebase
Belongs to Knowledgebase?
Yes
No
Within a path model in the co-variance map?
Yes
No
Provide Prediction
Has Quarantine Neighbours?
No
Yes
Laboratory Analysis
Update Quarantine Data
Local Model Exists?
No
Yes
Develop Path model
Update Knowledgebase
Learned Feature Space
Self-Learning Cycle

FIG. 8

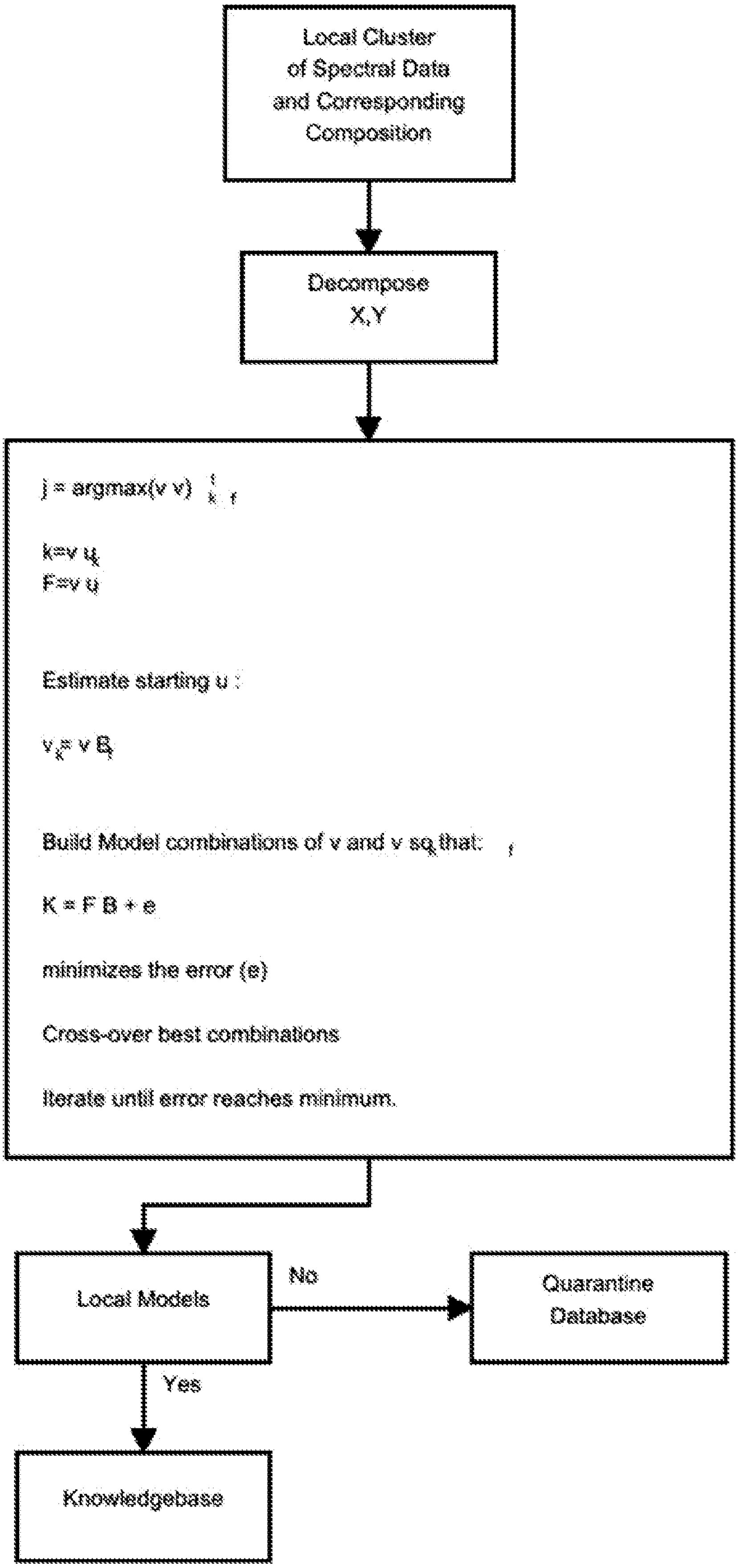
Local Cluster of Spectral Data and Corresponding Composition
Decompose X,Y
j = argmax(v v)
k=v u
F=v u
Estimate starting u :
v = v B
Build Model combinations of v and v so that:
K = F B + e
minimizes the error (e)
Cross-over best combinations
Iterate until error reaches minimum.
Local Models
No
Quarantine Database
Yes
Knowledgebase

FIG. 9

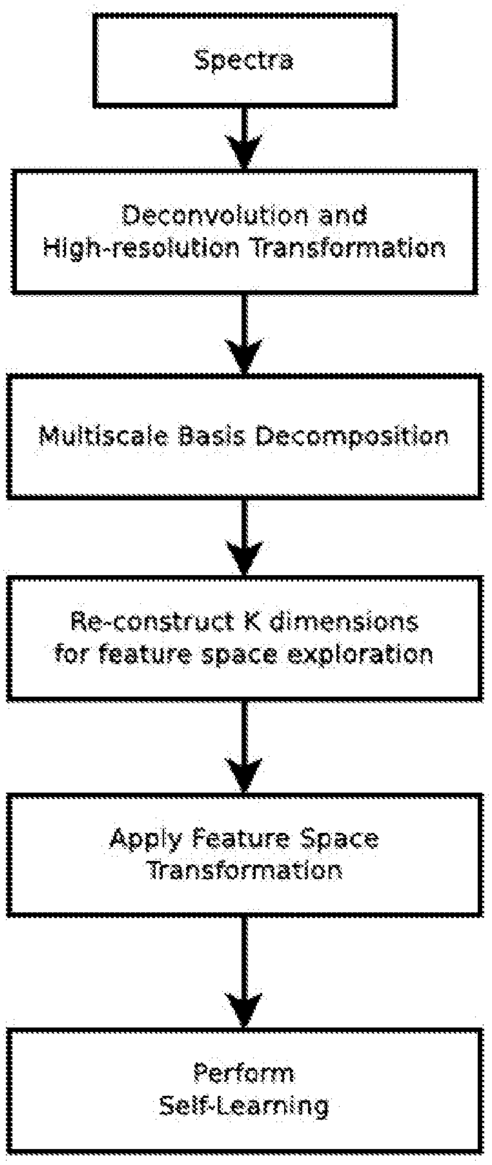
Spectra
Deconvolution and
High-resolution Transformation
Multiscale Basis Decomposition
Re-construct K dimensions
for feature space exploration
Apply Feature Space
Transformation
Perform
Self-Learning

FIG. 10

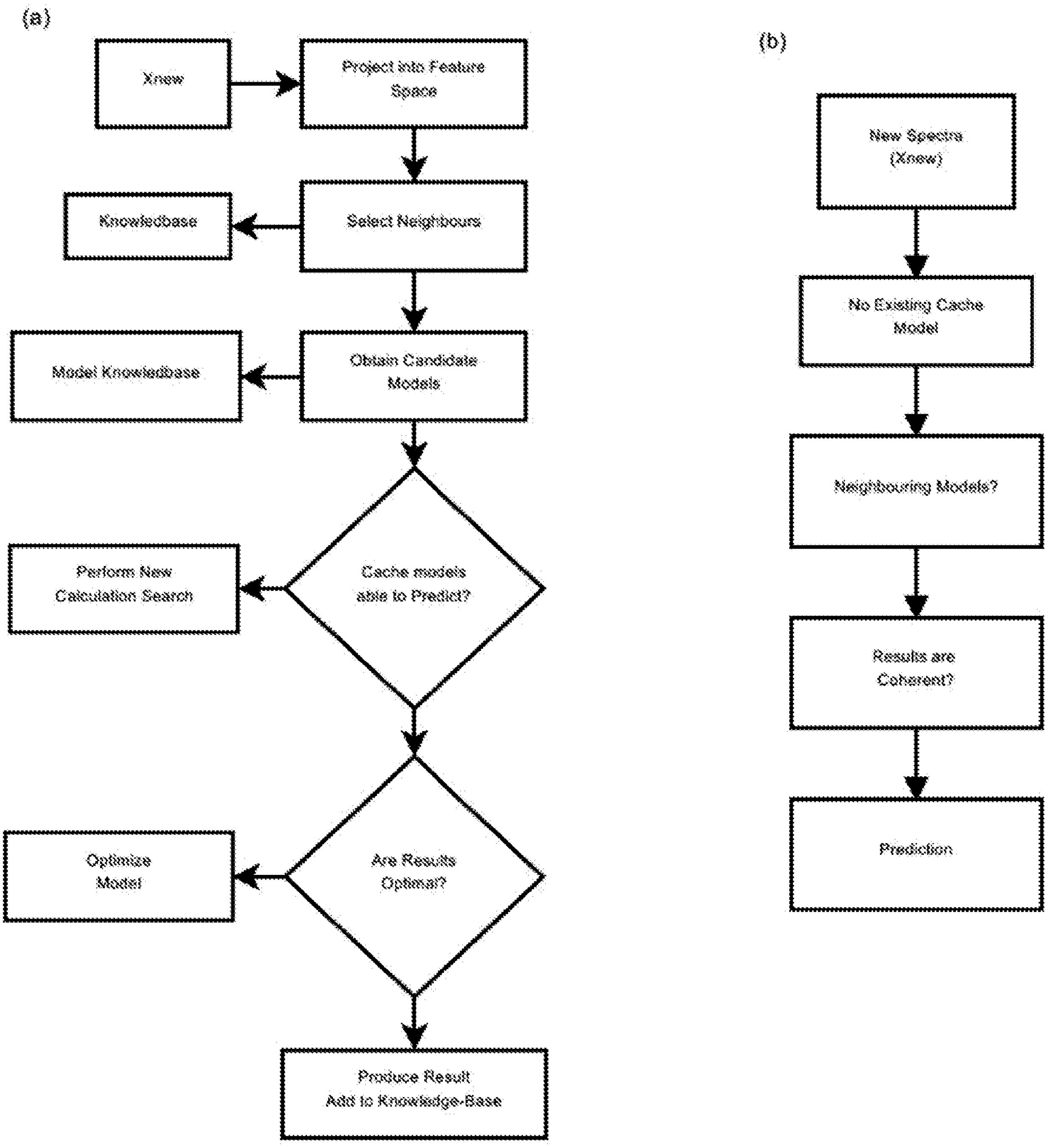
(a)
Xnew
Project into Feature Space
Knowledbase
Select Neighbours
Model Knowledbase
Obtain Candidate Models
Perform New Calculation Search
Cache models able to Predict?
Optimize Model
Are Results Optimal?
Produce Result Add to Knowledge-Base
(b)
New Spectra (Xnew)
No Existing Cache Model
Neighbouring Models?
Results are Coherent?
Prediction

FIG. 11

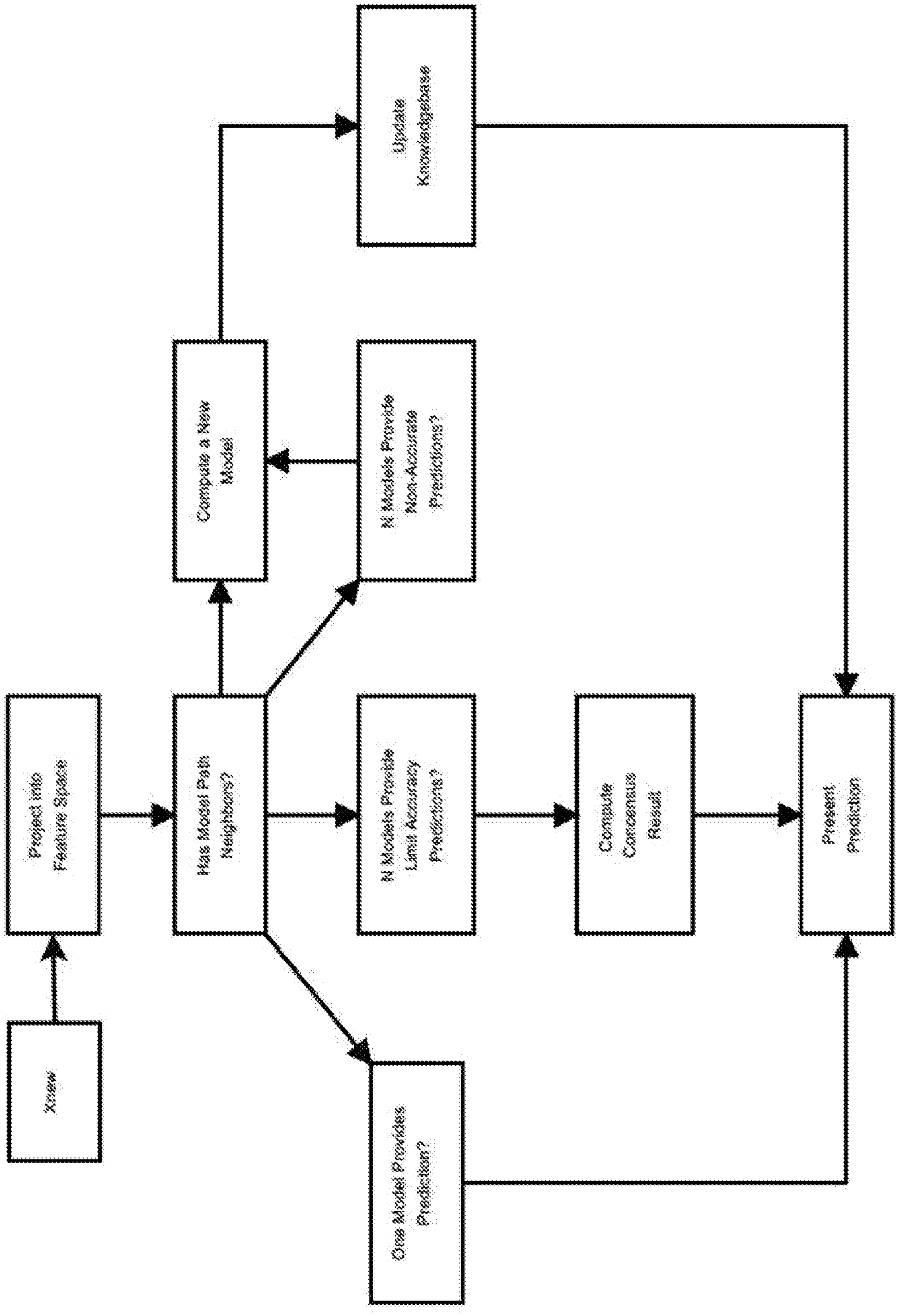
Xnew
Project into Feature Space
Has Model Path Neighbors?
N Models Provide Limit Accuracy Predictions?
Compute Consensus Result
Present Prediction
Compute a New Model
N Models Provide Non-Accurate Predictions?
Update Knowledgebase
One Model Provides Prediction?

FIG. 12

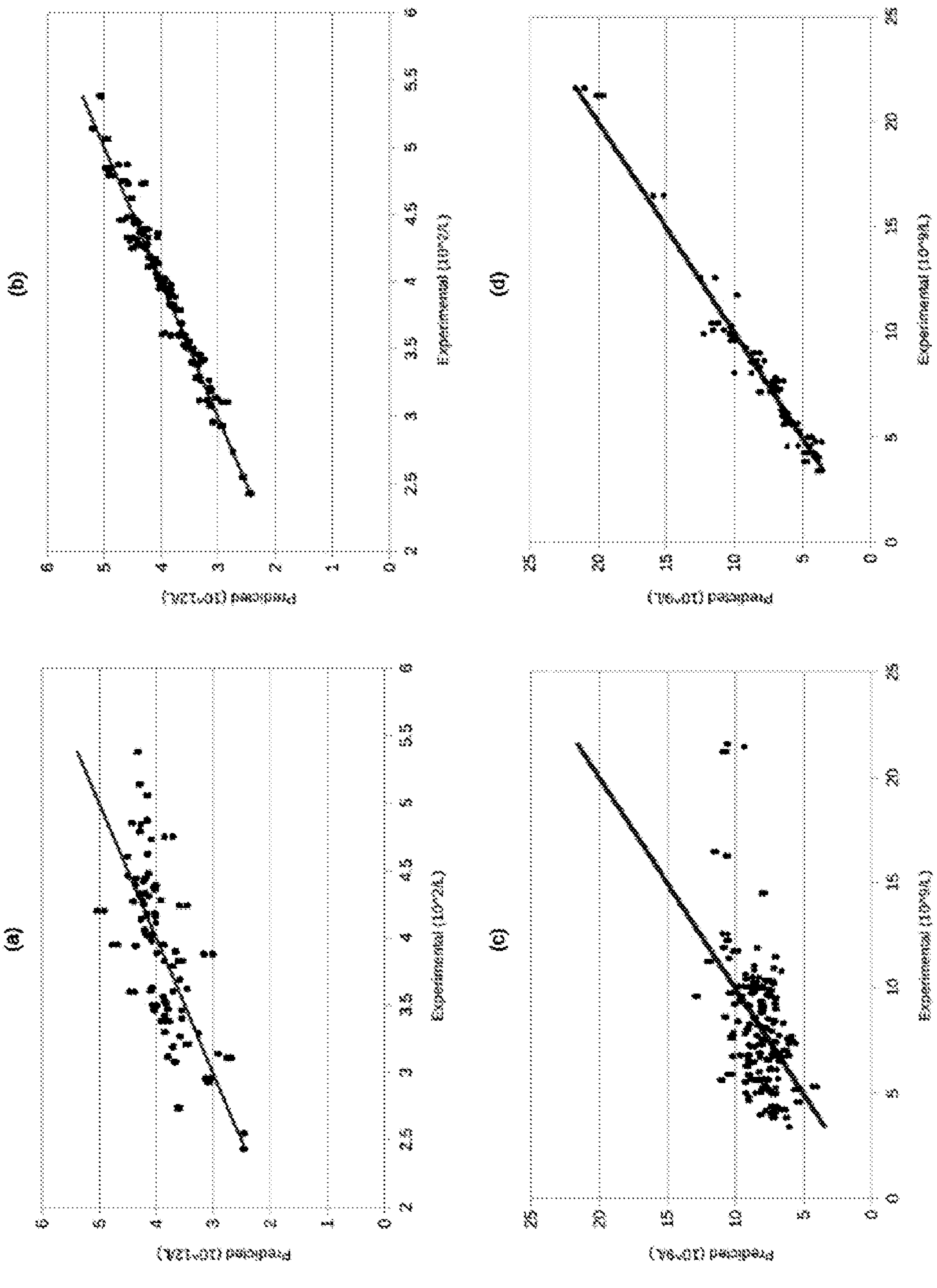


FIG. 13

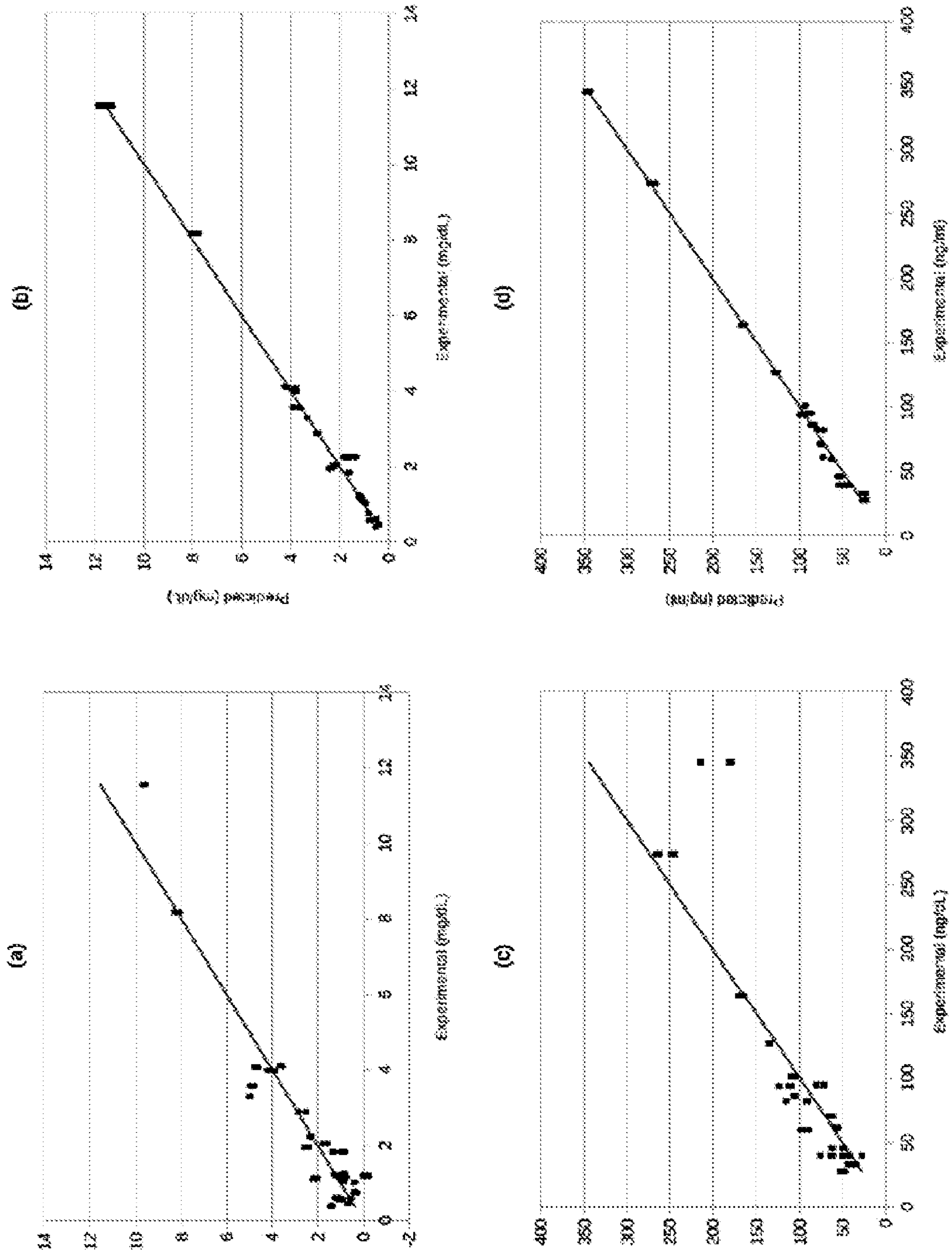
(a)
Predicted (mg/dL)
Experimental (mg/dL)
(b)
Predicted (mg/dL)
Experimental (mg/dL)
(c)
Predicted (ng/dL)
Experimental (ng/dL)
(d)
Predicted (ng/ml)
Experimental (ng/ml)

FIG. 14

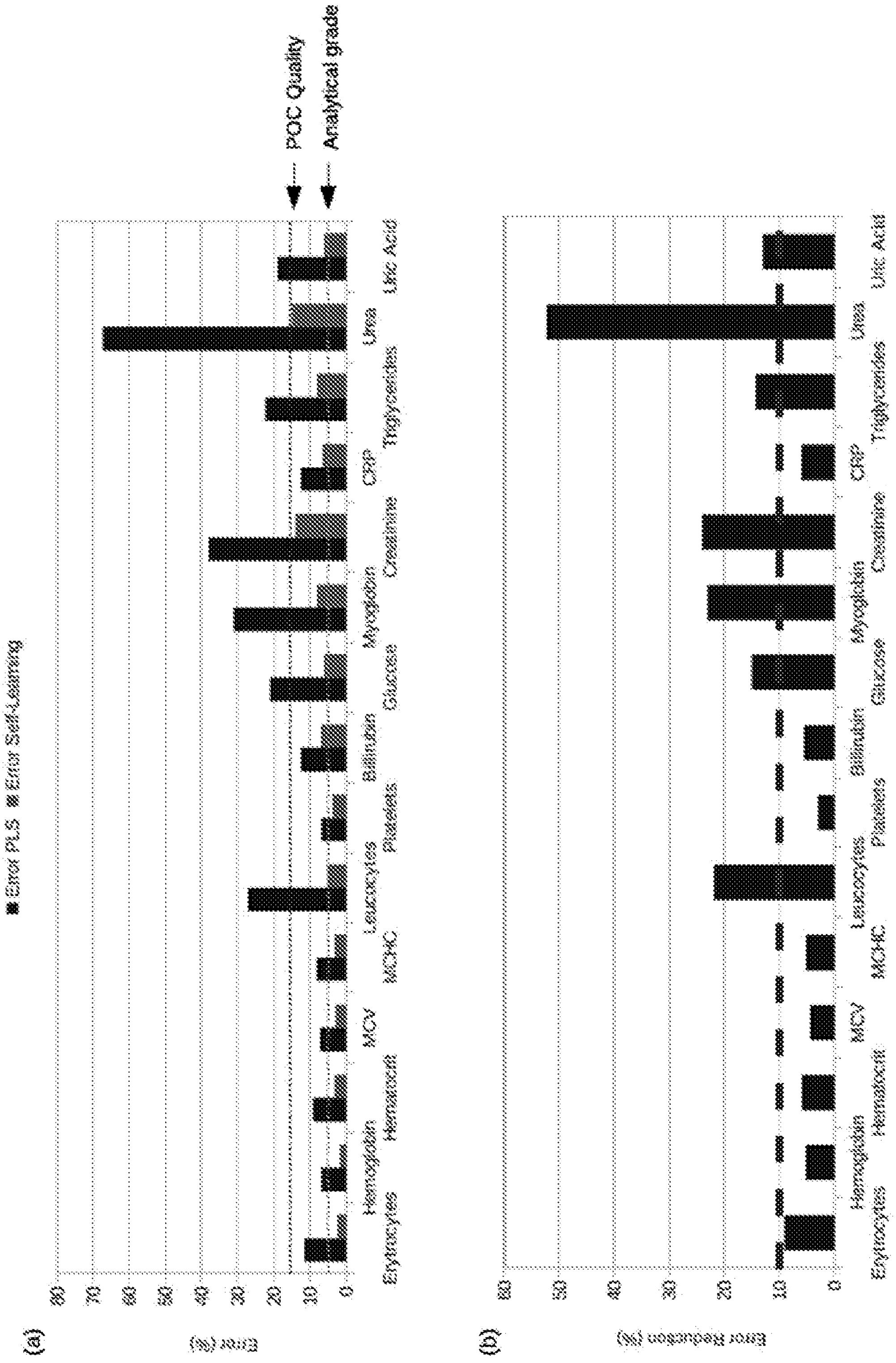
(a)
Error (%)
Error PLS
Error Self-Learning
POC Quality
Analytical grade
Erytrocytes
Hemoglobin
Hematocrit
MCV
MCHC
Leucocytes
Platelets
Bilirubin
Glucose
Myoglobin
Creatinine
CRP
Triglycerides
Urea
Uric Acid
(b)
Error Reduction (%)

FIG. 15

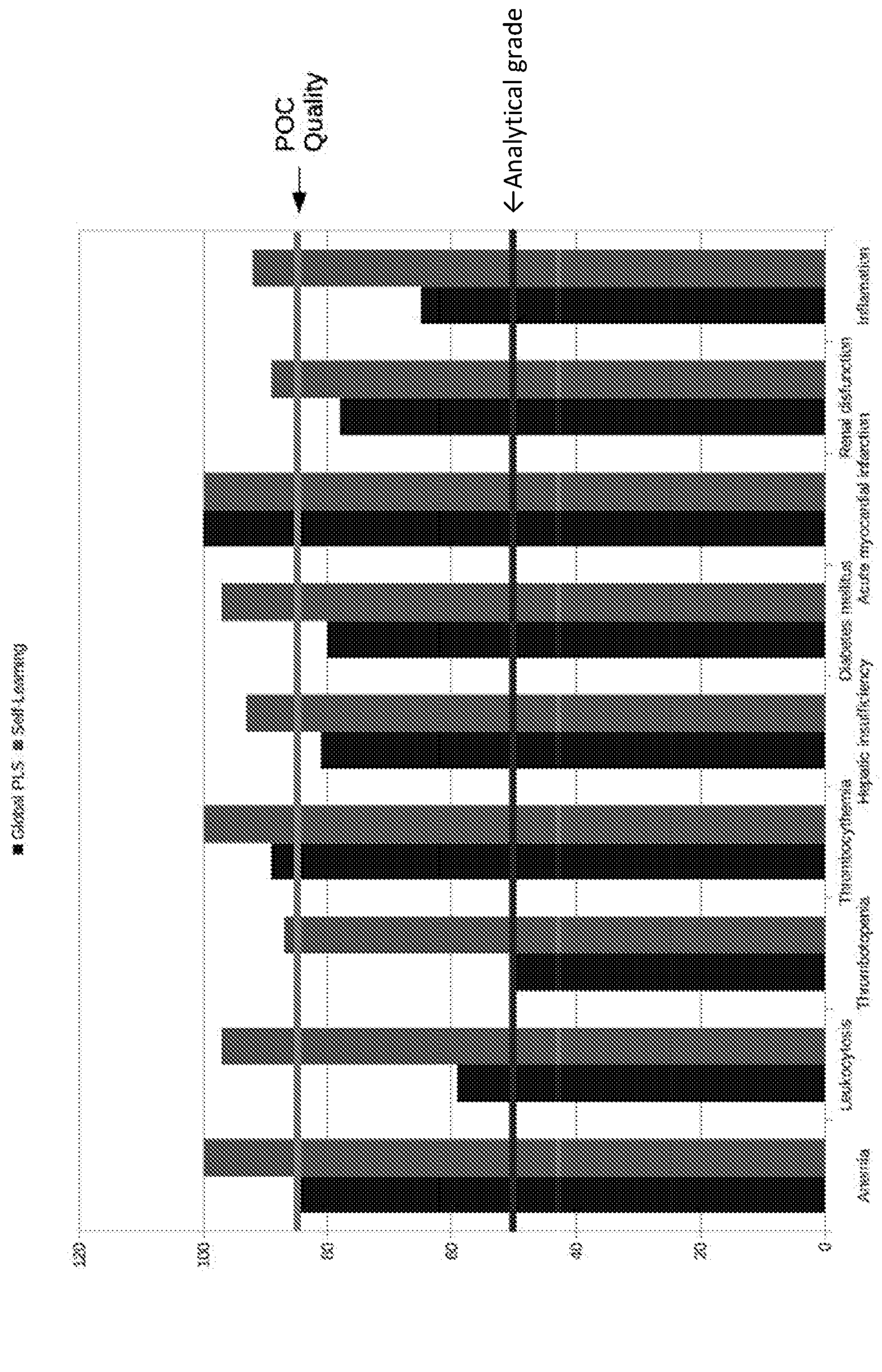
Global PLS
Self Learning
120
100
80
60
40
20
0
Anemia
Leukocytosis
Thrombocytopenia
Thrombocythemia
Hepatic insufficiency
Diabetes mellitus
Acute myocardial infarction
Renal disfunction
Inflammation
POC Quality
Analytical grade

FIG. 16

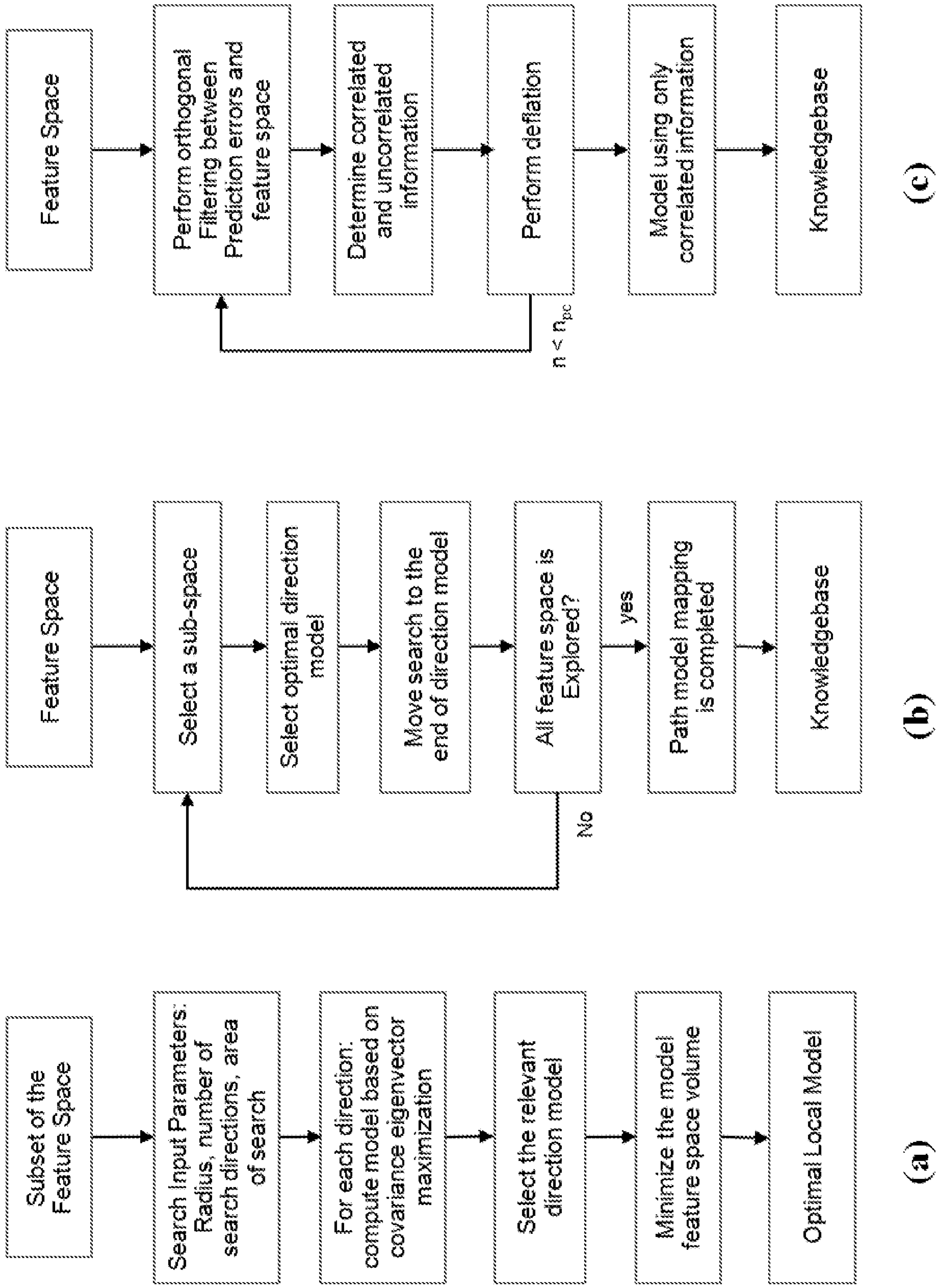
Subset of the Feature Space
Search Input Parameters: Radius, number of search directions, area of search
For each direction: compute model based on covariance eigenvector maximization
Select the relevant direction model
Minimize the model feature space volume
Optimal Local Model
(a)
Feature Space
Select a sub-space
Select optimal direction model
Move search to the end of direction model
All feature space is Explored?
yes
No
Path model mapping is completed
Knowledgebase
(b)
Feature Space
Perform orthogonal Filtering between Prediction errors and feature space
Determine correlated and uncorrelated information
Perform deflation
n < n_pc
Model using only correlated information
Knowledgebase
(c)

Fig. 17

**Algorithm 1** Local Geometry and Sub-space identification

**Step A.** Direction finding

**Require:** $\mathbf{U} \in \mathbf{F} \wedge \mathbf{U} = \mathbf{xV}$; where **V** is the basis of **F**

**Ensure:** $j = argmax(\mathbf{X}^t\mathbf{Y})_{local}$

```
r ← Radius                         %Assign search radius
d ← Directions                     %Assign number of search directions
v ← Volume                         %Assign initial volume of search directions
e_max ← Error                      %Assign alowable error of search direcions
S ← ComputeGeometry(r, d, v, U)    % S is a sub-space of F
FindDirections ← TRUE
while FindDirections==TRUE do
  for s in S do
    [e, npc, s] ← relevantPLS(Y, S)    %Obtain local optimum training set (s) by re-dimension of v
                                       %that minimizes error (e) and eigenvectors (nPC)
    if (e < e_max) and (npc < npc_max) then
      S ← [S|s]; E ← [E|e]; nPC ← [nPC|npc]    %Store relevant directions
      else
      s ← NewSearchDirection(s)                %Change search direction
      S ← [S|s]; E ← [E|e]; nPC ← [nPC|npc]    %Store relevant directions
    end if
  end for
  if (max(E) < e_max) and (max(nPC) < npc_max) then
    FindDirections ← FALSE             %Sub-space was identified
  end if
end while
Step B. Minimize sub-space convex hull volume
{C1, C2, C3} ← GenerateHullSimplex(C)    % Generate 3 convex hulls for simplex optimization
while max(E) < e_max2 do
  for S in C do
    [e, npc, s] ← relevantPLS(Y, S) % Obtain respective convex hull model
  end for
  {C1,C2} <- SelectMin(E) % Eliminate worst convex hull model
  C <- SimplexMove(C1,C2)
end while
Output: Best sub-space S ∈ F for predicting y given a spectra x
```

**Algorithm 2** Feature space mapping - knowledbase of quantification

**Require:** $\mathbf{U_i} \in \mathbf{F} \wedge \mathbf{U_i} = \mathbf{x_i V}$; where **V** is the basis of **F**

**Ensure:** $j = argmax(\mathbf{X}^t\mathbf{Y})_{local}$

```
r ← Radius                        %Assign search radius
d ← Directions                    %Assign number of search directions
v ← Volume                        %Assign initial volume of search direcions
e_max ← Error                     %Assign alowable error of search direcions
S ← ComputeGeometry(r,d,v,U)      % S is a sub-space of F
while NewSubSpace == TRUE do
  while NewSearchPossible == TRUE do
    Perform Algorithm 1
    NewSearchPossible ← GetNewSeachPoint()
    if NewSearchPossible ≠ NULL then
      S ← [S|s]; M ← [M|m]
      else
      NewSearchPossible ← NULL
    end if
  end while
  U_new ← SelectNewCoordinate()
  if U_new == NULL then
    NewSubSpace ← FALSE
  end if
end while
Output: Optimal sub-space mapping of S ∈ F for predicting y given by any spectra x
```

**Algorithm 3** Feature space mapping - knowledbase of classification

**Require:** $\mathbf{U_i} \in \mathbf{F} \wedge \mathbf{U_i} = \mathbf{x_i V}$; where **V** is the basis of **F**

**Ensure:** $j = argmax(\mathbf{X}^t\mathbf{L})_{local} \wedge \mathbf{L} = logit(\mathbf{P})$

```
r ← Radius                        %Assign search radius
d ← Directions                    %Assign number of search directions
v ← Volume                        %Assign initial volume of search direcions
e_max ← Error                     %Assign alowable error of search direcions
Clustering ← Error                %Define Clustering Criteria for a given P
C ← findConvexHulls(F,L)          %Find the convex hulls accross F
C ← CheckCLusterIndividuality(C)  %each convex hull must be independent
dv ← convexHullVolumeChange
for c in C do
  while ReShape == TRUE do
    u ← SelectVertice(c)          %Search starting coordinates
    S ← ComputeGeometry(r,d,v,u)  %Seach directions and geometry
    b ← Algorithm1(S)             % Boundary local subspace of u
    if b == NULL then
      l ← moveBoundary(c,dv)      % Move the inwards
      c ← findConvexHull(F,L,l)   %Determine the new convex hull
      Else
      ReShape ← FALSE
    end if
  end while
  [B|b] ← Algorithm2(c,b)         %Perform sequential sub-space identification of the convex hull boundary
end for
Output: Optimal sub-space mapping of L accross F for predicting p given by any spectra x
```

**Algorithm 4** Characterization of the local geometry

**Require:** $\mathbf{F}_1 \ldots \mathbf{F}_B \in \mathbf{F}, \mathbf{K}_1 \ldots \mathbf{K}_B \in \mathbf{K}; f(\mathbf{t},\mathbf{u}) = argmax(\mathbf{t}^t\mathbf{u})$

**Ensure:** $\|\mathbf{w}\| = 1, \|\mathbf{c}\| = 1, \mathbf{F} = \mathbf{T}\mathbf{W}^t, \mathbf{K} = \mathbf{U}\mathbf{C}^t; J = argmin\left\{PRESS \times \left[\frac{n_{pc}}{n} + \frac{n_{sel.vars}}{n_{vars}} + \frac{1}{corr(\mathbf{V},\mathbf{W})} + p - value\right]\right\}$

Given a search direction **D**:
**while** StableGroup = FALSE **do**
**for** $\mathbf{F}_i, \mathbf{K}_i$ in **D** **do**
$pc = 1$
**while** pcextraction=TRUE **do**
$\{\mathbf{w}, \Sigma, \mathbf{c}\} \leftarrow svd(\mathbf{K}^t\mathbf{F})$
**while** Converge = FALSE **do**
$\mathbf{t} \leftarrow \mathbf{F}\mathbf{w}/(\mathbf{w}^t\mathbf{w})$
$\mathbf{u} \leftarrow \mathbf{K}\mathbf{c}/(\mathbf{c}^t\mathbf{c})$
$\mathbf{w}^* \leftarrow \mathbf{F}^t\mathbf{u}/(\mathbf{u}^t\mathbf{u}); \mathbf{w}^* = \mathbf{w}^*/\|\mathbf{w}^*\|$
$\mathbf{c}^* \leftarrow \mathbf{K}^t\mathbf{c}/(\mathbf{c}^t\mathbf{c}); \mathbf{c}^* = \mathbf{c}^*/\|\mathbf{c}^*\|$
$\mathbf{e}_w \leftarrow \mathbf{w}_{j+1} - \mathbf{w}_j; \mathbf{e}_c \leftarrow \mathbf{c}_{j+1} - \mathbf{c}_j$
**if** $\mathbf{e}_w < threshould$ **and** $\mathbf{e}_c < threshould$ **then**
$Converge \leftarrow TRUE$
**end if**
**end while**
$\mathbf{p} = \mathbf{F}^t\mathbf{t}^*/(\mathbf{t}^{*t}\mathbf{t}^*); \mathbf{q} = \mathbf{K}^t\mathbf{t}^*/(\mathbf{t}^{*t}\mathbf{t}^*); [\mathbf{P}|\mathbf{p}] \leftarrow \mathbf{p}; [\mathbf{Q}|\mathbf{q}] \leftarrow \mathbf{q}$
$\mathbf{F} \leftarrow \mathbf{F} - \mathbf{t}\mathbf{p}^t; \mathbf{K} \leftarrow \mathbf{K} - \mathbf{t}\mathbf{q}^t$
$\beta_{pls} \leftarrow \mathbf{W}(\mathbf{P}^t\mathbf{W})^{-1}\mathbf{Q}$
$\hat{\mathbf{K}} \leftarrow \mathbf{F}\beta_{pls}$
$E_i^2 \leftarrow (\mathbf{K} - \hat{\mathbf{K}})^2$
**if** $E^2 \leftarrow decreases$ **then**
$pc \leftarrow pc + 1$
**else**
$pcextraction = FALSE$
**end if**
**end while**
$\mathbf{t}_i = \mathbf{T}_{\mathbf{c}}\beta_T$
$\{\mathbf{F}_i, \mathbf{K}_i\} \leftarrow SelectSamples(\mathbf{t}_i)$
**end for**
$\{\mathbf{F}, \mathbf{K}\} \leftarrow Recombine(\mathbf{F}, \mathbf{K})$
**if** $\{\mathbf{F}, \mathbf{K}\}$ is Stable **then**
StableGroup $\leftarrow$ TRUE
**end if**
**end while**
$\{\mathbf{F}, \mathbf{K}\} \leftarrow SelectVariables(\mathbf{F})$
**Output:** Optimal **F** and **F** for a given search direction **D**

**Algorithm 5** Sub-space information optimization using orthognal projections

**Require:** $\mathbf{F}_1 \ldots \mathbf{F}_n \in \mathbf{F}; \mathbf{K}_1 \ldots \mathbf{K}_n \in \mathbf{K}; f(\mathbf{t}, \mathbf{u}) = argmax(\mathbf{t}^t\mathbf{u})$

**Ensure:** $||\mathbf{w}|| = 1, ||\mathbf{c}|| = 1, \mathbf{F} = \mathbf{T}\mathbf{W}^t, \mathbf{K} = \mathbf{U}\mathbf{C}^t; J = argmin\left\{PRESS * \left[\frac{npc}{n} + \frac{n_{sel.vars}}{n_{vars}} + \frac{1}{cov(\mathbf{V},\mathbf{W})} + p - value\right]\right\}$

Given a search direction **D**:

Select **f**, **k** from **F**,**K** that maximizes $\mathbf{T}\mathbf{P}^t$ and minimizes $\mathbf{U}_o\mathbf{Q}_o^t$:

**while** $A < np$ **do**

$[\mathbf{c}_i, \Sigma_i, \mathbf{w}_i] = SVD(\mathbf{K}^t\mathbf{F}); \mathbf{w}_i = \mathbf{w}_i / ||\mathbf{w}_i||$

$\mathbf{t} = \mathbf{F}\mathbf{w}/\mathbf{w}^t\mathbf{w}; [\mathbf{T}|\mathbf{t}] \leftarrow \mathbf{t}$

$\mathbf{p} = \mathbf{F}^t\mathbf{t}/\mathbf{t}^t\mathbf{t}; [\mathbf{P}|\mathbf{p}] \leftarrow \mathbf{p}$

$\mathbf{q} = \mathbf{K}^t\mathbf{t}/\mathbf{t}^t\mathbf{t}; [\mathbf{Q}|\mathbf{q}] \leftarrow \mathbf{q}$

$\mathbf{E}_{f,i} = \mathbf{F}_i - \mathbf{T}_i\mathbf{P}_i^t$

$[\gamma_{o,i}, \Sigma_{o,k}, \mathbf{w}_{o,i}] = SVD(\mathbf{E}_{f,i}, \mathbf{T}_i); \mathbf{w}_{o,i} = \mathbf{w}_{o,i} / ||\mathbf{w}_{o,i}||$

$\mathbf{t}_o = \mathbf{F}_i\mathbf{w}_{o,i}$

$\mathbf{p}_o = \mathbf{F}^t\mathbf{t}_o/\mathbf{t}_o^t\mathbf{t}_o$ or $\mathbf{p}_o = \mathbf{p} - \mathbf{w}_o^t\mathbf{p}/\mathbf{w}_o^t\mathbf{w}_o; [\mathbf{P}_o|\mathbf{p}_o] \leftarrow \mathbf{p}_o$

$\mathbf{F}_{i+1} = \mathbf{F}_{i+1} - \mathbf{T}_i\mathbf{P}_i^t - \mathbf{T}_{o,i}\mathbf{P}_{o,i}^t$

$\mathbf{E}_{k,i} = \mathbf{K}_i - \mathbf{T}_i\mathbf{Q}_i^t$

$[\zeta_{o,i}, \Sigma_{o,k}, \mathbf{c}_{o,i}] = SVD(\mathbf{E}_{k,i}, \mathbf{T}_i); \mathbf{c}_{o,i} = \mathbf{c}_{o,i} / ||\mathbf{c}_{o,i}||$

$\mathbf{u}_o = \mathbf{K}_i\mathbf{c}_{o,i}$

$\mathbf{q} = \mathbf{K}^t\mathbf{u}_o/\mathbf{u}_o^t\mathbf{u}_o; [\mathbf{Q}_o|\mathbf{q}_o] \leftarrow \mathbf{q}_o$

$\mathbf{K}_{i+1} = \mathbf{K}_{i+1} - \mathbf{T}_i\mathbf{Q}_i^t - \mathbf{U}_{o,i}\mathbf{Q}_{o,i}^t$

**end while**

$\mathbf{F} = \mathbf{T}\mathbf{P}^t + \mathbf{T}_o\mathbf{P}_o^t$

$\mathbf{K} = \mathbf{T}\mathbf{Q}^t + \mathbf{U}_o\mathbf{Q}_o^t$

$\beta \leftarrow \mathbf{W}(\mathbf{P}^t\mathbf{W})^{-1}\mathbf{Q}$

**Output:** Optimal orthogonally filtered relationship $\mathbf{K} = \mathbf{F}\beta$ for a given search direction **D**

Table 1. Metrics for the characterization of the feature space

| Description | Formula | Objective |
|---|---|---|
| Statistical representation | n | number of data at a local direction |
| Latent Ratio | np/n | ratio of number of LV's vs number of local data |
| Complexity rate | 1/k | eigenvalue decrease ratio |
| Complexity | C = npc/(n · k) | complexity of local dataset |
| PRESS | $\sum(Y - \hat{Y})^2/(n-p)$ | predicted sum of squares |
| Coliniarity | $cov(\mathbf{v}, \mathbf{w})$ | colinearity of **F** and **K** eigenstructure |
| Model Variance | $R_F^2 = 1 - \sum \mathbf{E}_F^2 / \sum \mathbf{F}^2$ | Variance of **F** in model |
| Predicted Variance | $R_F^2 = 1 - \sum(\mathbf{T}\mathbf{Q}^t - \mathbf{K})^2 / \sum \mathbf{K}^2$ | Predicted model variance |
| Spectral Interference | $R_{Fo}^2 = \sum(\mathbf{T}_o\mathbf{P}_o^t)^2 / \sum \mathbf{F}^2$ | Ratio of orthogonal information in **F** |
| Quantification Interference | $R_{Ko}^2 = \sum(\mathbf{U}_o\mathbf{Q}_o^t)^2 / \sum \mathbf{K}^2$ | Ratio of orthogonal information in **K** |
| Co-variation | $R_{F,cov}^2 = \sum(\mathbf{T}\mathbf{P}^t)^2 / \sum \mathbf{F}^2$ | Ratio of co-variance between **F** – **K** |
| Co-variation | $R_{K,cov}^2 = \sum(\mathbf{T}\mathbf{Q}^t)^2 / \sum \mathbf{K}^2$ | Ratio of co-variance between **K** – **F** |
| Uncorrelated Information | $1 - R_{F,cov}^2$ | Uncorrelated information in **F** |
| Uncorrelated Information | $1 - R_{K,cov}^2$ | Uncorrelated information in **K** |

Table 2. Global PLS benchmarks versus self-learning methodology in blood samples

| | | Global PLS | | | Self-Learning Method | | |
|---|---|---|---|---|---|---|---|
| Parameter | Range | nLV | Error (%) | $R^2$ | $nLV^{(a)}$ | Error (%) | $R^2$ |
| Erytrocytes ($10^2/l$) | 2.0 – 5.5 | 7 | 11.5 | 0.3731 | 1 | 2.4 | 0.9889 |
| Hemoglobin ($g/dl$) | 6.5 – 16.0 | 8 | 6.9 | 0.8395 | 1 | 1.7 | 0.9897 |
| Hematocrit (%) | 20 – 45.4 | 7 | 9.2 | 0.8392 | 1 | 3.3 | 0.9846 |
| MCV (fL) | 75 – 120 | 7 | 7.2 | 0.8421 | 1 | 2.8 | 0.9872 |
| MCHC ($g/dl$) | 30 – 37 | 7 | 8.2 | 0.8312 | 1 | 3.1 | 0.9835 |
| Leucocytes ($10^9/l$) | 3 – 22 | 10 | 27.0 | 0.4512 | 1 | 5.2 | 0.9907 |
| Platelets ($10^9/l$) | 22 – 753 | 9 | 6.8 | 0.7911 | 1 | 3.8 | 0.9698 |
| Billirubin ($mg/dl$) | 0.25 – 1.5 | 6 | 12.5 | 0.9667 | 1 | 7.0 | 0.9838 |
| Glucose ($mg/dl$) | 60 – 370 | 10 | 21.0 | 0.7210 | 2 | 6.0 | 0.9865 |
| Myoglobin ($ng/ml$) | 27 – 345 | 8 | 31.0 | 0.9357 | 1 | 8.0 | 0.9930 |
| Creatinine ($mg/dl$) | 0.3 – 6.0 | 12 | 38.0 | 0.6134 | 3 | 14.0 | 0.9642 |
| CRP ($mg/dl$) | 3 – 14 | 8 | 12.5 | 0.9617 | 2 | 6.5 | 0.9834 |
| Triglycerides($mg/dl$) | 30 – 260 | 11 | 22.3 | 0.9156 | 3 | 8.0 | 0.9674 |
| Urea ($mg/dl$) | 10 – 140 | 8 | 67.3 | 0.8471 | 2 | 15.0 | 0.9768 |
| Uric Acid ($mg/dl$) | 1 – 8 | 7 | 19.0 | 0.7012 | 3 | 6.0 | 0.9479 |

(a) median number of LV's

Table 3. Classification benchmarks in blood samples: true vs false classifications

| | Global PLS | | | | Self-Learning Method | | | |
|---|---|---|---|---|---|---|---|---|
| Probability of relevance to: | True Positive (%) | True Negative (%) | False Positive (%) | False Negative (%) | True Positive (%) | True Negative (%) | False Positive (%) | False Negative (%) |
| Anemia | 45 | 40 | 4 | 11 | 53 | 47 | 0 | 0 |
| Leukocytosis | 21 | 38 | 6 | 15 | 48 | 50 | 0 | 2 |
| Thrombotopenia | 0 | 50 | 50 | 0 | 46 | 41 | 4 | 9 |
| Thrombocythemia | 39 | 50 | 11 | 0 | 50 | 50 | 0 | 0 |
| Hepatic insufficiency | 42 | 39 | 8 | 11 | 43 | 50 | 7 | 0 |
| Diabetes mellitus | 42 | 38 | 9 | 4 | 48 | 49 | 2 | 1 |
| Acute myocardial infarction | 50 | 50 | 0 | 0 | 50 | 50 | 0 | 0 |
| Renal disfunction | 37 | 41 | 13 | 9 | 43 | 46 | 8 | 3 |
| Inflamation | 50 | 15 | 0 | 35 | 50 | 42 | 0 | 8 |

(a) median number of LV's

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **A. PHATAK** ; **S. JONG**. The geometry of partial least squares. *Journal of Chemometrics*, 1997, vol. 11, 311-338
- **HUAN, S** ; **SONG** ; **K. YOU.** Trends in extreme learning machines: A review. *Neural Networks*, 2013, vol. 61, 32-48
- **L. RAMIREZ-LOPEZ** ; **T. BEHRENSA** ; **K. SCHMIDT**; **A. STEVENS** ; **J.A.M. DEMATTÊ** ; **T. SCHOLTEN**. The spectrum-based learner: A new local approach for modeling soil vis-nir spectra of complex datasets. *Geoderma*, 2013, vol. 195 - 196, 268-279
- **D.P. SOLOMATINE** ; **MASKEY. M.** ; **SHRESTHA. D.L.** Instance-based learning compared to other data-driven methods in hydrological forecasting. *Hydrol. Process*, 2008, vol. 22, 275-287
- **T. NAES** ; **T. ISAKSSON** ; **B. KOWALSKI**. Locally weighted regression and scatter correction for near-infrared reflectance data. *Anal. Chem.*, 1990, vol. 62 (7), 664-673
- **J.S. SHENK** ; **M.O. WESTERHAUS** ; **P.ERZAGHI**. Local prediction with near infrared multi-product databases. *Journal of Near Infrared Spectroscopy*, 1997, vol. 5, 223-232
- **T. FEARN** ; **A.M.C. DAVIES**. Locally-biased regression. *Journal of Near Infrared*, 2003, vol. 11 (6), 467-478
- **A.M.C DAVIES** ; **T.EARN.** Quantitative analysis via near infrared databases: comparison analysis using restructured nearest infrared and constituent datadeux (carnac-d). *Journal of Near Infrared*, 2003, vol. 14 (6), 403-411
- **F.GOGE** ; **R. JOFFRE** ; **C. JOLIVET** ; **I. ROSS** ; **L. RANJARD.** Optimization criteria in sample selection step of local regression for quantitative analysis of large soil nirs database. *Chemometrics and Intelligent Laboratory Systems*, 2012, vol. 110 (1), 168-176
- **L. RAMIREZ-LOPEZ** ; **T. BEHRENS** ; **K. SCHMIDT** ; **A. STEVENS** ; **J.A.M. DEMATTÊ** ; **T. SCHOLTEN**. The spectrum-based learner: a new local approach for modelling soil vis-nir spectra of complex datasets. *Geoderma*, 2013, vol. 195-196, 268-279
- **L. RAMIREZ-LOPEZ** ; **T.BEHRENS** ; **K.SCHMIDT** ; **R.A. VISCARRAROSSEL** ; **J.A.M. DEMATTÊ** ; **T. SCHOLTEN**. Distance and similarity-search metrics for use with soil vis nir spectra. *Geoderma*, 2013, vol. 199, 43-53
- **U.G. INDAHL**. The geometry of pls1 explained properly: 10 key notes on mathematical properties of and some alternative algorithmic approaches to pls1 modelling. *Journal of Chemometrics*, 2014, vol. 24, 168-180
- **R.J. PELL** ; **L.S. RAMOS** ; **R. MANNE**. The model space in partial least squares regression. *Journal of Chemometrics*, 2007, vol. 21, 165-172
- **S. WOLD** ; **M. HOYC** ; **H. MARTENS** ; **J. TRYGG** ; **F. WESTADE** ; **J. MACGREGOR** ; **B.M. WISE**. The pls model space revisited. *Journal of Chemometrics*, 2009, vol. 23, 67-68
- **R. ERGON**. Finding y-relevant part of x by use of pcr and plsr model reduction methods. *Journal of Chemometrics*, 2007, vol. 21, 537-546
- **R. ERGON**. Re-interpretation of nipals results solves plsr inconsistency problem. *Journal of Chemometrics*, 2009, vol. 23, 72-75
- **R.C. MARTINS** ; **V.V. LOPES** ; **P. VALENTÃO** ; **J.C.M.F. CARVALHO** ; **P. ISABEL** ; **M.T. AMARAL** ; **M.T. BATISTA** ; **P. B. ANDRADE** ; **B.M. SILVA**. Relevant principal component analysis applied to the characterisation of portuguese heather honey. *Natural Product Research*, 2007, vol. 22, 1560-1582